# FASCICULE DE BREVET EUROPEEN

(11) **EP 2 470 908 B1**
(45) Date de publication et mention de la délivrance du brevet: **19.06.2013**
(21) Numéro de dépôt: 10763762.1
(22) Date de dépôt: 27.08.2010
(51) Int. Cl.: G01N 33/569, C07K 14/20

(54) **PROTEINES UTILISEES POUR LE DIAGNOSTIC D'UNE BORRELIOSE DE LYME**
PROTEINE ZUR DIAGNOSE VON LYME-BORRELIOSE
PROTEINS USED FOR THE DIAGNOSIS OF LYME BORRELIOSIS

(30) Priorité: 28.08.2009 FR 0904094
(43) Date de publication de la demande: 04.07.2012
(73) Titulaire: Biomérieux, 69280 Marcy L'etoile (FR)
(72) Inventeur: LEVET, Lionel, 69380 Alix (FR); MEJAN-LETOURNEUR, Odile, F-69005 Lyon (FR)
(86) Numéro de dépôt international: PCT/FR2010/051787
(87) Numéro de publication internationale: WO 2011/023914

(56) Documents cités:
- US-A1- 2009 162 875
- US-B1- 6 475 492
- MARANGONI A ET AL: "Borrelia burgdorferi VlsE antigen for the serological diagnosis of Lyme borreliosis", EUROPEAN JOURNAL OF CLINICAL MICROBIOLOGY & INFECTIOUS DISEASES, SPRINGER, BERLIN, DE, vol. 27, no. 5, 16 janvier 2008 (2008-01-16), pages 349-354, XP019594724, ISSN: 1435-4373
- TJERNBERG I ET AL: "Antibody responses to borrelia IR6 peptide variants and the C6 peptide in Swedish patients with erythema migrans", INTERNATIONAL JOURNAL OF MEDICAL MICROBIOLOGY, URBAN UND FISCHER, DE LNKD- DOI:10.1016/J.IJMM.2008.10.006, vol. 299, no. 6, 1 août 2009 (2009-08-01), pages 439-446, XP026497760, ISSN: 1438-4221 [extrait le 2009-01-09]

### SEQUENCE LISTING

<110> bioMérieux
<120> Protéines utilisées pour le diagnostic d'une Borréliose de Lyme
<130> Lyme VlsE PCT
<160> 47
<170> PatentIn version 3.3
<210> 1
   <211> 274
   <212> PRT
   <213> Borrelia sp.
<400> 1
   Lys Asn Asn Val Gly Gly Asp Asp Lys Lys Asp Thr Ala Ala Ser Ile
   1               5                   10                  15
   Phe Tyr Gln Ser Ile Ile Asn Leu Gly Asn Gly Phe Ile Glu Val Phe
               20                  25                  30
   Asn Ala Phe Ser Gly Leu Val Ala Asp Ala Phe Ser Lys Ala Asp Pro
           35                  40                  45
   Lys Lys Ser Asp Val Lys Thr Tyr Phe Asp Ser Ile Thr Lys Thr Leu
       50                  55                  60
   Lys Asp Thr Lys Thr Lys Leu Glu Asp Ile Ser Lys Glu Lys Thr Gly
   65                  70                  75                  80
   Gly Glu Lys Thr Pro Ala Val Glu Gly Ile Ala Glu Val Val Lys Thr
                   85                  90                  95
   Val Gly Glu Trp Leu Asp Gly Leu Ile Lys Ala Ala Glu Gly Gly Gly
               100                 105                 110
   Lys Ala Ala Asp Gly Gly Gly Ser Asp Lys Ile Gly Asn Val Ala Ala
           115                 120                 125
   Gly Gly Gly Ala Gly Ala Asp Lys Glu Ser Val Asn Gly Ile Ala Gly
       130                 135                 140
   Ala Ile Lys Gly Ile Val Glu Ala Ala Lys Lys Val Glu Gly Val Lys
   145                 150                 155                 160
   Phe Ala Pro Lys Ala Ala Ala Asp Ala Ala Ala Ala Asp Gly Asn Lys
                   165                 170                 175
   Lys Ala Gly Lys Leu Phe Gly Thr Ala Ala Gly Ala Asp Ala Gly Asp
               180                 185                 190
   Val Lys Asp Ala Ala Ala Ala Val Gly Ala Val Ser Gly Glu Gln Ile
   195 200 205
   Leu Asn Ala Ile Val Thr Ala Ala Gly Gln Ala Gly Gln Ala Gly Lys
   210 215 220
   Lys Ala Asp Glu Ala Lys Asn Ala Ile Glu Ala Ala Ile Gly Ala Ala
   225 230 235 240
   Gly Asp Ala Asp Phe Gly Asp Asp Ile Lys Lys Lys Asn Asp Gln Ile
   245 250 255
   Ala Ala Ala Leu Val Leu Arg Gly Val Ala Lys Asp Gly Lys Phe Ala
   260 265 270
   Gly Ala
<210> 2
   <211> 280
   <212> PRT
   <213> Borrelia sp.
<400> 2
   Lys Asn Ser Ala Gly Asp Ile Ser Asn Lys Ser Asp Glu Asn Asp Pro
   1 5 10 15
   Thr Thr Leu Phe Tyr Gln Ser Ile Ile Lys Leu Gly Asn Gly Phe Leu
   20 25 30
   Glu Val Phe Thr Ser Phe Gly Gly Met Val Ala Asp Ala Phe Gly Ala
   35 40 45
   Lys Trp Glu Ala Lys Lys Ser Thr Ile Lys Thr Tyr Phe Asp Thr Met
   50 55 60
   Ser Gln Lys Leu Glu Glu Thr Lys Lys Gly Leu Glu Lys Leu Ala Asn
   65 70 75 80
   Asn Gly Glu Glu Ser Glu Ser Glu Asn Lys Ile Gly Asp Ala Val Ala
   85 90 95
   Ser Thr Ile Lys Glu Val Gly Glu Trp Leu Thr Glu Met Ala Thr Ala
   100 105 110
   Ala Gly Gly Ala Ala Lys Val Ala Asp Ser Gly Gly Asp Glu Ile Gly
   115 120 125
   Lys Val Glu Asn Ala Gly Ala Asn Ala Asn Lys Gly Asp Lys Thr Ser
       130                 135                 140
   Val Asn Gly Ile Ala Lys Gly Ile Lys Ala Ile Val Gly Val Ala Lys
   145                 150                 155                 160
   Lys Ala Gly Val Lys Trp Glu Pro Ala Ala Ala Ala Glu Ala Gly Asp
                   165                 170                 175
   Ala Asn Gly Asn Lys Asn Ala Gly Lys Leu Phe Ala Thr Gly Gly Gln
               180                 185                 190
   Gly Asp Ala Ala Ala Gly Lys Glu Ala Ala Leu Ala Val Ser Gly Val
           195                 200                 205
   Ser Gly Asp Gln Ile Leu Asn Ala Ile Val Thr Asp Ala Glu Gly Asp
       210                 215                 220
   Lys Asn Gly Val Ala Thr Ala Asn Ala Thr Asn Ser Ile Asp Ala Ala
   225                 230                 235                 240
   Ile Gly Ala Asp Gly Asp Asn Gly Ala Ser Gly Phe Asp Ala Met Lys
                   245                 250                 255
   Lys Lys Asn Asp Lys Ile Ala Ala Ala Ile Val Leu Arg Gly Met Ala
               260                 265                 270
   Lys Asp Gly Lys Phe Ala Val Lys
           275                 280
<210> 3
   <211> 284
   <212> PRT
   <213> Borrelia sp.
<400> 3
   Lys Asn Asn Ala Glu Leu Ala Glu Ala Glu Ala Lys Asn Gln Ser Ala
   1               5                   10                  15
   Lys Asp Phe Tyr His Ala Ile Ile Lys Leu Gly Tyr Gly Phe Val Asp
               20                  25                  30
   Val Phe Asn Ala Ile Gly Gly Leu Val Ser Asp Val Phe Tyr Lys Ala
           35                  40                  45
   Asp Pro Lys Lys Ser Asp Val Lys Asn Tyr Phe Asp Ser Ile Ala Ser
       50                  55                  60
   Ile Leu Lys Glu Thr Gln Thr Lys Leu Asp Ala Leu Ser Lys Glu Gln
   65                  70                  75                  80
   Gly Gly Gly Asp Gly Gly Thr Gln Val Val Asp Ala Ala Lys Lys Ala
   85 90 95
   Gly Glu Trp Ile Lys Glu Met His Lys Ala Val Glu Asp Thr Ala Lys
   100 105 110
   Ala Gly Gly Glu Gly Gly Ser Glu Ser Ile Ala Asn Val Ala Ala Gly
   115 120 125
   Gly Gly Gly Asn Asp Gly Ala Gly Ala Lys Ala Asp Val Asn Ser Val
   130 135 140
   Thr Gly Ile Ala Lys Gly Met Lys Ala Ile Val Asp Ala Ala Gly Lys
   145 150 155 160
   Ala Gly Val Glu Leu Lys Pro Ala Ala Ala Gly Gly Ala Ala Ala Asn
   165 170 175
   Asp Ala Gly Lys Leu Phe Ala Ser Gly Ala Asn Ala Asn Ala Ala Ala
   180 185 190
   Asn Ala Asp Asp Ala Glu Gly Ala Ala Glu Ala Ala Gly Lys Ala Val
   195 200 205
   Ser Ala Val Ser Gly Asp Gln Ile Leu Lys Ala Ile Val Asp Ala Ala
   210 215 220
   Gly Ala Thr Ala Gly Lys Lys Ala Asn Glu Ala Thr Asn Ala Val Glu
   225 230 235 240
   Ala Ala Ile Gly Asp Asp Asn Ala Gly Gln Ala Gly Ala Ala Phe Ala
   245 250 255
   Ala Gly Met Gln Asn Lys Asn Asp Gln Ile Ala Ala Ala Ile Val Leu
   260 265 270
   Arg Gly Leu Ala Lys Ser Gly Lys Phe Ala Asn Glu
   275 280
<210> 4
   <211> 279
   <212> PRT
   <213> Borrelia sp.
<400> 4
   Lys Asn Asn Ala Val Gly Lys Gly Asn Asp Asp Lys Asp Ser Val Lys
   1 5 10 15
   Thr Phe Tyr Glu Ser Ile Ile Asn Leu Gly Asn Gly Phe Ile Asp Val
               20                  25                  30
   Phe Asn Ala Phe Ser Gly Leu Val Ala Asp Thr Phe Phe Lys Ser Asp
           35                  40                  45
   Pro Lys Lys Ser Asp Val Lys Thr Tyr Phe Glu Ser Ile Ser Ser Thr
       50                  55                  60
   Leu Lys Ala Thr Lys Gly Lys Leu Asp Glu Leu Val Ser Ala Lys Lys
   65                  70                  75                  80
   Gly Glu Gly Gly Ser Val Lys Ala Ser Val Glu Ser Ala Val Asp Glu
                   85                  90                  95
   Val Ser Lys Trp Leu Glu Glu Met Ile Lys Ala Ala Glu Glu Ala Ala
               100                 105                 110
   Lys Val Gly Gly Thr Gly Gly Asp Gly Lys Ile Gly Asp Ser Ala Ala
           115                 120                 125
   Asn His Gly Ala Lys Ala Asp Lys Asp Ser Val Lys Gly Ile Ala Lys
       130                 135                 140
   Gly Ile Lys Gly Ile Val Asp Ala Ala Gly Lys Ala Leu Gly Glu Lys
   145                 150                 155                 160
   Gly Ala Leu Lys Asp Val Lys Ala Ala Ala Asp Asp Glu Ala Asn Ala
                   165                 170                 175
   Asp Ala Gly Lys Leu Phe Ala Gly Asn Ala Asn Ala Ala Val Gly Ala
               180                 185                 190
   Ala Ala Asp Ile Ala Lys Ala Ala Gly Ala Val Thr Ala Val Ser Gly
           195                 200                 205
   Glu Gln Ile Leu Lys Ala Ile Val Glu Ala Ala Gly Asp Pro Ala Asn
       210                 215                 220
   Gln Ala Gly Lys Lys Ala Glu Glu Ala Lys Asn Pro Ile Ala Ala Ala
   225                 230                 235                 240
   Ile Gly Thr Asp Asp Asp Asn Gly Ala Ala Phe Lys Asp Glu Met Lys
                   245                 250                 255
   Lys Ser Asp Lys Ile Ala Ala Ala Ile Val Leu Arg Gly Val Ala Lys
               260                 265                 270
   Asp Gly Lys Phe Ala Val Lys
   275
<210> 5
   <211> 279
   <212> PRT
   <213> Borrelia sp.
<400> 5
   Lys Ser Gln Val Ala Asp Lys Asp Asp Pro Thr Asn Lys Phe Tyr Gln
   1               5                   10                  15
   Ser Val Ile Gln Leu Gly Asn Gly Phe Leu Asp Val Phe Thr Ser Phe
               20                  25                  30
   Gly Gly Leu Val Ala Glu Ala Phe Gly Phe Lys Ser Asp Pro Lys Lys
           35                  40                  45
   Ser Asp Val Lys Thr Tyr Phe Thr Thr Val Ala Ala Lys Leu Glu Lys
       50                  55                  60
   Thr Lys Thr Asp Leu Asn Ser Leu Pro Lys Glu Lys Ser Asp Ile Ser
   65                  70                  75                  80
   Ser Thr Thr Gly Lys Pro Asp Ser Thr Gly Ser Val Gly Thr Ala Val
                   85                  90                  95
   Glu Gly Ala Ile Lys Glu Val Ser Glu Leu Leu Asp Lys Leu Val Lys
               100                 105                 110
   Ala Val Lys Thr Ala Glu Gly Ala Ser Ser Gly Thr Ala Ala Ile Gly
           115                 120                 125
   Glu Val Val Ala Asp Ala Asp Ala Ala Lys Val Ala Asp Lys Ala Ser
       130                 135                 140
   Val Lys Gly Ile Ala Lys Gly Ile Lys Glu Ile Val Glu Ala Ala Gly
   145                 150                 155                 160
   Gly Ser Glu Lys Leu Lys Ala Val Ala Ala Ala Lys Gly Glu Asn Asn
                   165                 170                 175
   Lys Gly Ala Gly Lys Leu Phe Gly Lys Ala Gly Ala Ala Ala His Gly
               180                 185                 190
   Asp Ser Glu Ala Ala Ser Lys Ala Ala Gly Ala Val Ser Ala Val Ser
           195                 200                 205
   Gly Glu Gln Ile Leu Ser Ala Ile Val Thr Ala Ala Asp Ala Ala Glu
       210                 215                 220
   Gln Asp Gly Lys Lys Pro Glu Glu Ala Lys Asn Pro Ile Ala Ala Ala
   225                 230                 235                 240
   Ile Gly Asp Lys Asp Gly Gly Ala Glu Phe Gly Gln Asp Glu Met Lys
                   245                 250                 255
   Lys Asp Asp Gln Ile Ala Ala Ala Ile Ala Leu Arg Gly Met Ala Lys
               260                 265                 270
   Asp Gly Lys Phe Ala Val Lys
           275
<210> 6
   <211> 25
   <212> PRT
   <213> Borrelia sp.
<400> 6
   Met Lys Lys Asp Asp Gln Ile Ala Ala Ala Ile Ala Leu Arg Gly Met
   1               5                   10                  15
   Ala Lys Asp Gly Lys Phe Ala Val Lys
               20                  25
<210> 7
   <211> 17
   <212> PRT
   <213> Borrelia sp.
<400> 7
   Ile Val Ala Ala Ile Val Leu Arg Gly Val Ala Lys Ser Gly Lys Phe
   1               5                   10                  15
   Ala
<210> 8
   <211> 25
   <212> PRT
   <213> Borrelia sp.
<400> 8
   Met Lys Lys Asp Asp Gln Ile Ala Ala Ala Met Val Leu Arg Gly Met
   1               5                   10                  15
   Ala Lys Asp Gly Gln Phe Ala Leu Lys
               20                  25
<210> 9
   <211> 7
   <212> PRT
   <213> Borrelia sp.
<400> 9
   Asp Gly Glu Lys Glu Lys Ala
   1 5
<210> 10
   <211> 6
   <212> PRT
   <213> Artificial
<220>
   <223> Tag His
<400> 10
   His His His His His His
   1 5
<210> 11
   <211> 18
   <212> DNA
   <213> Artificial
<220>
   <223> DNA Tag His
<400> 11
   catcatcatc atcatcat 18
<210> 12
   <211> 18
   <212> DNA
   <213> Artificial
<220>
   <223> DNA Tag His
<400> 12
   catcatcatc atcatcac 18
<210> 13
   <211> 18
   <212> DNA
   <213> Artificial
<220>
   <223> DNA Tag His
<400> 13
   catcatcacc accatcat 18
<210> 14
   <211> 4
   <212> PRT
   <213> Artificial
<220>
   <223> aa+
<400> 14
   Met Arg Gly Ser
   1
<210> 15
   <211> 12
   <212> DNA
   <213> Artificial
<220>
   <223> DNA aa+
<400> 15
   atgaggggat cc 12
<210> 16
   <211> 8
   <212> PRT
   <213> Artificial
<220>
   <223> Tag His 3
<400> 16
   His His His His His His His His
   1 5
<210> 17
   <211> 24
   <212> DNA
   <213> Artificial
<220>
   <223> DNA Tag His
<400> 17
   catcatcatc atcatcatca tcat 24
<210> 18
   <211> 2
   <212> PRT
   <213> Artificial
<220>
   <223> aa+ 1
<400> 18
   Met Gly
   1
<210> 19
   <211> 6
   <212> DNA
   <213> Artificial
<220>
   <223> DNA aa+ 1
<400> 19
   atgggc 6
<210> 20
   <211> 348
   <212> PRT
   <213> Borrelia sp.
<400> 20
   Lys Asn Asn Val Gly Gly Asp Asp Lys Lys Asp Thr Ala Ala Ser Ile
   1               5                   10                  15
   Phe Tyr Gln Ser Ile Ile Asn Leu Gly Asn Gly Phe Ile Glu Val Phe
               20                  25                  30
   Asn Ala Phe Ser Gly Leu Val Ala Asp Ala Phe Ser Lys Ala Asp Pro
           35                  40                  45
   Lys Lys Ser Asp Val Lys Thr Tyr Phe Asp Ser Ile Thr Lys Thr Leu
       50                  55                  60
   Lys Asp Thr Lys Thr Lys Leu Glu Asp Ile Ser Lys Glu Lys Thr Gly
   65                  70                  75                  80
   Gly Glu Lys Thr Pro Ala Val Glu Gly Ile Ala Glu Val Val Lys Thr
                   85                  90                  95
   Val Gly Glu Trp Leu Asp Gly Leu Ile Lys Ala Ala Glu Gly Gly Gly
               100                 105                 110
   Lys Ala Ala Asp Gly Gly Gly Ser Asp Lys Ile Gly Asn Val Ala Ala
           115                 120                 125
   Gly Gly Gly Ala Gly Ala Asp Lys Glu Ser Val Asn Gly Ile Ala Gly
       130                 135                 140
   Ala Ile Lys Gly Ile Val Glu Ala Ala Lys Lys Val Glu Gly Val Lys
   145                 150                 155                 160
   Phe Ala Pro Lys Ala Ala Ala Asp Ala Ala Ala Ala Asp Gly Asn Lys
                   165                 170                 175
   Lys Ala Gly Lys Leu Phe Gly Thr Ala Ala Gly Ala Asp Ala Gly Asp
               180                 185                 190
   Val Lys Asp Ala Ala Ala Ala Val Gly Ala Val Ser Gly Glu Gln Ile
           195                 200                 205
   Leu Asn Ala Ile Val Thr Ala Ala Gly Gln Ala Gly Gln Ala Gly Lys
       210                 215                 220
   Lys Ala Asp Glu Ala Lys Asn Ala Ile Glu Ala Ala Ile Gly Ala Ala
   225                 230                 235                 240
   Gly Asp Ala Asp Phe Gly Asp Asp Ile Lys Lys Lys Asn Asp Gln Ile
                   245                 250                 255
   Ala Ala Ala Leu Val Leu Arg Gly Val Ala Lys Asp Gly Lys Phe Ala
               260                 265                 270
   Gly Ala Met Lys Lys Asp Asp Gln Ile Ala Ala Ala Ile Ala Leu Arg
           275                 280                 285
   Gly Met Ala Lys Asp Gly Lys Phe Ala Val Lys Asp Gly Glu Lys Glu
       290                 295                 300
   Lys Ala Ile Val Ala Ala Ile Val Leu Arg Gly Val Ala Lys Ser Gly
   305                 310                 315                 320
   Lys Phe Ala Met Lys Lys Asp Asp Gln Ile Ala Ala Ala Met Val Leu
                   325                 330                 335
   Arg Gly Met Ala Lys Asp Gly Gln Phe Ala Leu Lys
               340                 345
<210> 21
   <211> 358
   <212> PRT
   <213> Borrelia sp.
<400> 21
   Met Arg Gly Ser His His His His His His Lys Asn Asn Val Gly Gly
   1               5                   10                  15
   Asp Asp Lys Lys Asp Thr Ala Ala Ser Ile Phe Tyr Gln Ser Ile Ile
               20                  25                  30
   Asn Leu Gly Asn Gly Phe Ile Glu Val Phe Asn Ala Phe Ser Gly Leu
           35                  40                  45
   Val Ala Asp Ala Phe Ser Lys Ala Asp Pro Lys Lys Ser Asp Val Lys
       50                  55                  60
   Thr Tyr Phe Asp Ser Ile Thr Lys Thr Leu Lys Asp Thr Lys Thr Lys
   65                  70                  75                  80
   Leu Glu Asp Ile Ser Lys Glu Lys Thr Gly Gly Glu Lys Thr Pro Ala
                   85                  90                  95
   Val Glu Gly Ile Ala Glu Val Val Lys Thr Val Gly Glu Trp Leu Asp
   100 105 110
   Gly Leu Ile Lys Ala Ala Glu Gly Gly Gly Lys Ala Ala Asp Gly Gly
   115 120 125
   Gly Ser Asp Lys Ile Gly Asn Val Ala Ala Gly Gly Gly Ala Gly Ala
   130 135 140
   Asp Lys Glu Ser Val Asn Gly Ile Ala Gly Ala Ile Lys Gly Ile Val
   145 150 155 160
   Glu Ala Ala Lys Lys Val Glu Gly Val Lys Phe Ala Pro Lys Ala Ala
   165 170 175
   Ala Asp Ala Ala Ala Ala Asp Gly Asn Lys Lys Ala Gly Lys Leu Phe
   180 185 190
   Gly Thr Ala Ala Gly Ala Asp Ala Gly Asp Val Lys Asp Ala Ala Ala
   195 200 205
   Ala Val Gly Ala Val Ser Gly Glu Gln Ile Leu Asn Ala Ile Val Thr
   210 215 220
   Ala Ala Gly Gln Ala Gly Gln Ala Gly Lys Lys Ala Asp Glu Ala Lys
   225 230 235 240
   Asn Ala Ile Glu Ala Ala Ile Gly Ala Ala Gly Asp Ala Asp Phe Gly
   245 250 255
   Asp Asp Ile Lys Lys Lys Asn Asp Gln Ile Ala Ala Ala Leu Val Leu
   260 265 270
   Arg Gly Val Ala Lys Asp Gly Lys Phe Ala Gly Ala Met Lys Lys Asp
   275 280 285
   Asp Gln Ile Ala Ala Ala Ile Ala Leu Arg Gly Met Ala Lys Asp Gly
   290 295 300
   Lys Phe Ala Val Lys Asp Gly Glu Lys Glu Lys Ala Ile Val Ala Ala
   305 310 315 320
   Ile Val Leu Arg Gly Val Ala Lys Ser Gly Lys Phe Ala Met Lys Lys
   325 330 335
   Asp Asp Gln Ile Ala Ala Ala Met Val Leu Arg Gly Met Ala Lys Asp
   340 345 350
   Gly Gln Phe Ala Leu Lys
   355
<210> 22
   <211> 1077
   <212> DNA
   <213> Borrelia sp.
<400> 22
   atgaggggat cccaccacca ccatcatcat aaaaataatg tcggcggcga tgacaaaaaa      60
   gatactgcgg ccagcatctt ctaccagtct attattaacc tgggtaacgg gttcattgaa     120
   gtgtttaatg ccttttccgg gctggtggcc gacgcgttta gcaaagcaga tccgaaaaaa     180
   tcagatgtca aaacttactt cgattcgatc acgaaaacac tgaaagatac caaaactaag     240
   ctggaagata ttagcaaaga aaaaacgggc ggcgaaaaaa cgccagccgt tgaaggtatc     300
   gccgaagtcg tgaaaaccgt gggagagtgg ctggatggcc tgattaaagc ggcggaaggg     360
   ggcggcaaag cggcggatgg tggcggttcg gacaaaattg ggaatgtcgc tgcaggcggc     420
   ggcgcgggcg ccgacaagga aagtgtgaat ggaatcgcag gtgccattaa aggtatcgtg     480
   gaagctgcaa aaaaggtgga aggtgtgaaa ttcgccccga aagctgcggc ggatgcagcc     540
   gccgctgatg gtaacaaaaa agcaggcaaa ctgtttggta ccgcggcggg cgcagacgcg     600
   ggagacgtga aagatgcagc cgctgcggta ggggccgtga gcggtgaaca gattctgaat     660
   gcgattgtta cggcggcggg ccaggcaggc caggcgggga aaaaagctga tgaagcaaaa     720
   aatgcgattg aagctgccat tggtgcggct ggcgatgcgg attttggtga cgacattaaa     780
   aagaaaaacg atcaaattgc ggcggcgctg gttctgcgcg gagttgctaa agacggcaaa     840
   tttgccggcg ctatgaagaa agacgaccaa atcgcggcag ccattgcgct gcgcggcatg     900
   gcgaaagacg gcaaatttgc ggtgaaagat ggcgaaaaag aaaaagcgat tgtggcggcg     960
   atcgttctgc gcggtgttgc gaaaagcggt aaattcgcga tgaaaaaaga tgatcagatc    1020
   gccgcagcga tggttctgcg tggtatggcc aaagatggtc agtttgccct gaaataa       1077
<210> 23
   <211> 357
   <212> PRT
   <213> Borrelia sp.
<400> 23
   Met Gly His His His His His His His His Lys Asn Asn Val Gly Gly
   1               5                   10                  15
   Asp Asp Lys Lys Asp Thr Ala Ala Ser Ile Phe Tyr Gln Ser Ile Ile
               20                  25                  30
   Asn Leu Gly Asn Gly Phe Ile Glu Val Phe Asn Ala Phe Ser Gly Leu
           35                  40                  45
   Val Ala Asp Ala Phe Ser Lys Ala Asp Pro Lys Lys Ser Asp Val Lys
       50                  55                  60
   Thr Tyr Phe Asp Ser Ile Thr Lys Thr Leu Lys Asp Thr Lys Thr Lys
   65                  70                  75                  80
   Leu Glu Asp Ile Ser Lys Glu Lys Thr Gly Gly Glu Lys Thr Pro Ala
                   85                  90                  95
   Val Glu Gly Ile Ala Glu Val Val Lys Thr Val Gly Glu Trp Leu Asp
               100                 105                 110
   Gly Leu Ile Lys Ala Ala Glu Gly Gly Gly Lys Ala Ala Asp Gly Gly
           115                 120                 125
   Gly Ser Asp Lys Ile Gly Asn Val Ala Ala Gly Gly Gly Ala Gly Ala
       130                 135                 140
   Asp Lys Glu Ser Val Asn Gly Ile Ala Gly Ala Ile Lys Gly Ile Val
   145                 150                 155                 160
   Glu Ala Ala Lys Lys Val Glu Gly Val Lys Phe Ala Pro Lys Ala Ala
                   165                 170                 175
   Ala Asp Ala Ala Ala Ala Asp Gly Asn Lys Lys Ala Gly Lys Leu Phe
               180                 185                 190
   Gly Thr Ala Ala Gly Ala Asp Ala Gly Asp Val Lys Asp Ala Ala Ala
           195                 200                 205
   Ala Val Gly Ala Val Ser Gly Glu Gln Ile Leu Asn Ala Ile Val Thr
       210                 215                 220
   Ala Gly Gln Ala Gly Gln Ala Gly Lys Lys Ala Asp Glu Ala Lys Asn
   225                 230                 235                 240
   Ala Ile Glu Ala Ala Ile Gly Ala Ala Gly Asp Ala Asp Phe Gly Asp
                   245                 250                 255
   Asp Ile Lys Lys Lys Asn Asp Gln Ile Ala Ala Ala Leu Val Leu Arg
               260                 265                 270
   Gly Val Ala Lys Asp Gly Lys Phe Ala Gly Ala Met Lys Lys Asp Asp
           275                 280                 285
   Gln Ile Ala Ala Ala Ile Ala Leu Arg Gly Met Ala Lys Asp Gly Lys
       290                 295                 300
   Phe Ala Val Lys Asp Gly Glu Lys Glu Lys Ala Ile Val Ala Ala Ile
   305 310 315 320
   Val Leu Arg Gly Val Ala Lys Ser Gly Lys Phe Ala Met Lys Lys Asp
   325 330 335
   Asp Gln Ile Ala Ala Ala Met Val Leu Arg Gly Met Ala Lys Asp Gly
   340 345 350
   Gln Phe Ala Leu Lys
   355
<210> 24
   <211> 1074
   <212> DNA
   <213> Borrelia sp.
<400> 24
   atgggccatc atcatcatca tcatcatcat aaaaacaacg tgggcggcga tgataaaaaa 60
   gataccgcgg cgagcatttt ttatcagagc attattaacc tgggcaacgg ctttattgaa 120
   gtgtttaacg cgtttagcgg cctggtggcg gatgcgttta gcaaagcgga tccgaaaaaa 180
   agcgatgtga aaacctattt tgatagcatt accaaaaccc tgaaagatac caaaaccaaa 240
   ctggaagata ttagcaaaga aaaaaccggc ggcgaaaaaa ccccggcggt ggaaggcatt 300
   gcggaagtgg tgaaaaccgt gggcgaatgg ctggatggcc tgattaaagc ggcggaaggc 360
   ggcggcaaag cggcggatgg cggcggcagc gataaaattg gcaacgtggc ggcgggcggc 420
   ggcgcgggcg cggataaaga aagcgtgaac ggcattgcgg gcgcgattaa aggcattgtg 480
   gaagcggcga aaaaagtgga aggcgtgaaa tttgcgccga aagcggcggc ggatgcggcg 540
   gcggcggatg gcaacaaaaa agcgggcaaa ctgtttggca ccgcggcggg cgcggatgcg 600
   ggcgatgtga aagatgcggc ggcggcggtg ggcgcggtga gcggcgaaca gattctgaac 660
   gcgattgtga ccgcgggcca ggcgggccag gcgggcaaaa aagcggatga agcgaaaaac 720
   gcgattgaag cggcgattgg cgcggcgggc gatgcggatt ttggcgatga tattaaaaaa 780
   aaaaacgatc agattgcggc ggcgctggtg ctgcgcggcg tggcgaaaga tggcaaattt 840
   gcgggcgcga tgaaaaaaga tgatcagatt gcggcggcga ttgcgctgcg cggcatggcg 900
   aaagatggca aatttgcggt gaaagatggc gaaaaagaaa aagcgattgt ggcggcgatt 960
   gtgctgcgcg gcgtggcgaa aagcggcaaa tttgcgatga aaaaagatga tcagattgcg 1020
   gcggcgatgg tgctgcgcgg catggcgaaa gatggccagt ttgcgctgaa ataa 1074
<210> 25
   <211> 149
   <212> PRT
   <213> Borrelia sp.
<400> 25
   Ser Leu Thr Gly Lys Ala Arg Leu Glu Ser Ser Val Lys Asp Ile Thr
   1               5                   10                  15
   Asn Glu Ile Glu Lys Ala Ile Lys Glu Ala Glu Asp Ala Gly Val Lys
               20                  25                  30
   Thr Asp Ala Phe Thr Glu Thr Gln Thr Gly Gly Lys Val Ala Gly Pro
           35                  40                  45
   Lys Ile Arg Ala Ala Lys Ile Arg Val Ala Asp Leu Thr Ile Lys Phe
       50                  55                  60
   Leu Glu Ala Thr Glu Glu Glu Thr Ile Thr Phe Lys Glu Asn Gly Ala
   65                  70                  75                  80
   Gly Glu Asp Glu Phe Ser Gly Ile Tyr Asp Leu Ile Leu Asn Ala Ala
                   85                  90                  95
   Lys Ala Val Glu Lys Ile Gly Met Lys Asp Met Thr Lys Thr Val Glu
               100                 105                 110
   Glu Ala Ala Lys Glu Asn Pro Lys Thr Thr Ala Asn Gly Ile Ile Glu
           115                 120                 125
   Ile Val Lys Val Met Lys Ala Lys Val Glu Asn Ile Lys Glu Lys Gln
       130                 135                 140
   Thr Lys Asn Gln Lys
   145
<210> 26
   <211> 211
   <212> PRT
   <213> Borrelia sp.
<400> 26
   Lys Lys Asn Thr Leu Ser Ala Ile Leu Met Thr Leu Phe Leu Phe Ile
   1               5                   10                  15
   Ser Cys Asn Asn Ser Gly Lys Gly Gly Asp Ser Ala Ser Thr Asn Pro
               20                  25                  30
   Ala Asp Glu Ser Ala Lys Gly Pro Asn Leu Thr Glu Ile Ser Lys Lys
           35                  40                  45
   Ile Thr Asp Ser Asn Ala Phe Val Leu Ala Val Lys Glu Val Glu Thr
       50                  55                  60
   Leu Val Leu Ser Ile Asp Glu Leu Ala Lys Lys Ala Ile Gly Gln Lys
   65                  70                  75                  80
   Ile Asp Asn Asn Asn Gly Leu Ala Ala Leu Asn Asn Gln Asn Gly Ser
                   85                  90                  95
   Leu Leu Ala Gly Ala Tyr Ala Ile Ser Thr Leu Ile Thr Glu Lys Leu
               100                 105                 110
   Ser Lys Leu Lys Asn Leu Glu Glu Leu Lys Thr Glu Ile Ala Lys Ala
           115                 120                 125
   Lys Lys Cys Ser Glu Glu Phe Thr Asn Lys Leu Lys Ser Gly His Ala
       130                 135                 140
   Asp Leu Gly Lys Gln Asp Ala Thr Asp Asp His Ala Lys Ala Ala Ile
   145                 150                 155                 160
   Leu Lys Thr His Ala Thr Thr Asp Lys Gly Ala Lys Glu Phe Lys Asp
                   165                 170                 175
   Leu Phe Glu Ser Val Glu Gly Leu Leu Lys Ala Ala Gln Val Ala Leu
               180                 185                 190
   Thr Asn Ser Val Lys Glu Leu Thr Ser Pro Val Val Ala Glu Ser Pro
           195                 200                 205
   Lys Lys Pro
       210
<210> 27
   <211> 164
   <212> PRT
   <213> Borrelia sp.
<400> 27
   Thr Gly Ala Thr Lys Ile Arg Leu Glu Arg Ser Ala Lys Asp Ile Thr
   1               5                   10                  15
   Asp Glu Ile Asp Ala Ile Lys Lys Asp Ala Ala Leu Lys Gly Val Asn
               20                  25                  30
   Phe Asp Ala Phe Lys Asp Lys Lys Thr Gly Ser Gly Val Ser Glu Asn
           35                  40                  45
   Pro Phe Ile Leu Glu Ala Lys Val Arg Ala Thr Thr Val Ala Glu Lys
       50                  55                  60
   Phe Val Ile Ala Ile Glu Glu Glu Ala Thr Lys Leu Lys Glu Thr Gly
   65                  70                  75                  80
   Ser Ser Gly Glu Phe Ser Ala Met Tyr Asp Leu Met Phe Glu Val Ser
                   85                  90                  95
   Lys Pro Leu Gln Lys Leu Gly Ile Gln Glu Met Thr Lys Thr Val Ser
               100                 105                 110
   Asp Ala Ala Glu Glu Asn Pro Pro Thr Thr Ala Gln Gly Val Leu Glu
           115                 120                 125
   Ile Ala Lys Lys Met Arg Glu Lys Leu Gln Arg Val His Thr Lys Asn
       130                 135                 140
   Tyr Cys Thr Leu Lys Lys Lys Glu Asn Ser Thr Phe Thr Asp Glu Lys
   145                 150                 155                 160
   Cys Lys Asn Asn
<210> 28
   <211> 185
   <212> PRT
   <213> Borrelia sp.
<400> 28
   Asn Thr Ser Ala Asn Ser Ala Asp Glu Ser Val Lys Gly Pro Asn Leu
   1               5                   10                  15
   Thr Glu Ile Ser Lys Lys Ile Thr Asp Ser Asn Ala Val Leu Leu Ala
               20                  25                  30
   Val Lys Glu Val Glu Ala Leu Leu Ser Ser Ile Asp Glu Ile Ala Ala
           35                  40                  45
   Lys Ala Ile Gly Lys Lys Ile His Gln Asn Asn Gly Leu Asp Thr Glu
       50                  55                  60
   Asn Asn His Asn Gly Ser Leu Leu Ala Gly Ala Tyr Ala Ile Ser Thr
   65                  70                  75                  80
   Leu Ile Lys Gln Lys Leu Asp Gly Leu Lys Asn Glu Gly Leu Lys Glu
                   85                  90                  95
   Lys Ile Asp Ala Ala Lys Lys Cys Ser Glu Thr Phe Thr Asn Lys Leu
               100                 105                 110
   Lys Glu Lys His Thr Asp Ser Phe Gly Lys Glu Gly Val Thr Asp Ala
           115                 120                 125
   Asp Ala Lys Glu Ala Ile Leu Lys Thr Asn Gly Thr Lys Thr Lys Gly
       130                 135                 140
   Ala Glu Glu Leu Gly Lys Leu Phe Glu Ser Val Glu Val Leu Ser Lys
   145                 150                 155                 160
   Ala Ala Lys Glu Met Leu Ala Asn Ser Val Lys Glu Leu Thr Ser Pro
                   165                 170                 175
   Val Val Ala Glu Ser Pro Lys Lys Pro
               180                 185
<210> 29
   <211> 162
   <212> PRT
   <213> Borrelia sp.
<400> 29
   Thr Gly Glu Thr Lys Ile Arg Leu Glu Ser Ser Ala Gln Glu Ile Lys
   1               5                   10                  15
   Asp Glu Ile Asn Lys Ile Lys Ala Asn Ala Lys Lys Glu Gly Val Lys
               20                  25                  30
   Phe Glu Ala Phe Thr Asp Lys Gln Thr Gly Ser Lys Val Ser Glu Lys
           35                  40                  45
   Pro Glu Phe Ile Leu Lys Ala Lys Ile Lys Ala Ile Gln Val Ala Glu
       50                  55                  60
   Lys Phe Val Lys Ala Ile Lys Glu Glu Ala Glu Lys Leu Lys Lys Ser
   65                  70                  75                  80
   Gly Ser Ser Gly Ala Phe Ser Ala Met Tyr Asp Leu Met Leu Asp Val
                   85                  90                  95
   Ser Lys Pro Leu Glu Glu Ile Gly Ile Gln Lys Met Thr Gly Thr Val
               100                 105                 110
   Thr Lys Glu Ala Glu Lys Thr Pro Pro Thr Thr Ala Glu Gly Ile Leu
           115                 120                 125
   Ala Ile Ala Gln Ala Met Glu Glu Lys Leu Asn Asn Val Asn Lys Lys
       130                 135                 140
   Gln Gln Asp Ala Leu Lys Asn Leu Glu Glu Lys Ala Asn Thr Ala Ala
   145                 150                 155                 160
   Thr Thr
<210> 30
   <211> 154
   <212> PRT
   <213> Borrelia sp.
<400> 30
   Ser Gly Thr Gly Lys Ala Arg Leu Glu Ser Ser Val Lys Asp Ile Thr
   1               5                   10                  15
   Asp Glu Ile Asp Lys Ala Ile Lys Glu Ala Ile Ala Asp Gly Val Lys
               20                  25                  30
   Leu Asn Glu Leu Glu Glu Asn Lys Thr Gly Ala Lys Lys Gly Gly Pro
           35                  40                  45
   Gln Ile Arg Asp Ala Lys Ile Arg Val Ile Asn Leu Ser Val Lys Phe
       50                  55                  60
   Leu Lys Glu Ile Glu Glu Glu Ala Asn Ile Leu Lys Asp Asn Val Gly
   65                  70                  75                  80
   Met Asn Lys Val Asp Lys Asp Gln Leu Leu Lys Asp Met Tyr Asp Leu
                   85                  90                  95
   Met Leu Asn Ala Ala Gly Ser Leu Gln Lys Leu Gly Leu Gln Glu Met
               100                 105                 110
   Ile Lys Thr Val Thr Gln Ala Ala Glu Lys Thr Pro Pro Thr Thr Val
           115                 120                 125
   Glu Gly Ile Leu Met Ile Ala Asn Thr Ile Glu Asp Lys Leu Lys Lys
       130                 135                 140
   Ile Lys Gly Lys Gln Glu Thr Asn Lys Lys
   145                 150
<210> 31
   <211> 176
   <212> PRT
   <213> Borrelia sp.
<400> 31
   Asp Glu Ser Ala Lys Gly Pro Asn Leu Thr Val Ile Ser Lys Lys Ile
   1               5                   10                  15
   Thr Asp Ser Asn Ala Phe Leu Leu Ala Val Lys Glu Val Glu Ala Leu
               20                  25                  30
   Leu Ser Ser Ile Asp Glu Leu Ser Lys Ala Ile Gly Lys Lys Ile Lys
   35 40 45
   Asn Asp Gly Thr Leu Asp Asn Glu Ala Asn Arg Asn Glu Ser Leu Ile
   50 55 60
   Ala Gly Ala Tyr Glu Ile Ser Lys Leu Ile Thr Gln Lys Leu Ser Val
   65 70 75 80
   Leu Asn Ser Glu Glu Leu Lys Glu Lys Ile Lys Glu Ala Lys Asp Cys
   85 90 95
   Ser Glu Lys Phe Thr Thr Lys Leu Lys Asp Ser His Ala Glu Leu Gly
   100 105 110
   Ile Gln Ser Val Gln Asp Asp Asn Ala Lys Lys Ala Ile Leu Lys Thr
   115 120 125
   His Gly Thr Lys Asp Lys Gly Ala Lys Glu Leu Glu Glu Leu Phe Lys
   130 135 140
   Ser Leu Glu Ser Leu Ser Lys Ala Ala Gln Ala Ala Leu Thr Asn Ser
   145 150 155 160
   Val Lys Glu Leu Thr Asn Pro Val Val Ala Glu Ser Pro Lys Lys Pro
   165 170 175
<210> 32
   <211> 361
   <212> PRT
   <213> Borrelia sp.
<400> 32
   Met Ser Leu Thr Gly Lys Ala Arg Leu Glu Ser Ser Val Lys Asp Ile
   1               5                   10                  15
   Thr Asn Glu Ile Glu Lys Ala Ile Lys Glu Ala Glu Asp Ala Gly Val
               20                  25                  30
   Lys Thr Asp Ala Phe Thr Glu Thr Gln Thr Gly Gly Lys Val Ala Gly
           35                  40                  45
   Pro Lys Ile Arg Ala Ala Lys Ile Arg Val Ala Asp Leu Thr Ile Lys
       50                  55                  60
   Phe Leu Glu Ala Thr Glu Glu Glu Thr Ile Thr Phe Lys Glu Asn Gly
   65                  70                  75                  80
   Ala Gly Glu Asp Glu Phe Ser Gly Ile Tyr Asp Leu Ile Leu Asn Ala
                   85                  90                  95
   Ala Lys Ala Val Glu Lys Ile Gly Met Lys Asp Met Thr Lys Thr Val
               100                 105                 110
   Glu Glu Ala Ala Lys Glu Asn Pro Lys Thr Thr Ala Asn Gly Ile Ile
           115                 120                 125
   Glu Ile Val Lys Val Met Lys Ala Lys Val Glu Asn Ile Lys Glu Lys
       130                 135                 140
   Gln Thr Lys Asn Gln Lys Lys Lys Asn Thr Leu Ser Ala Ile Leu Met
   145                 150                 155                 160
   Thr Leu Phe Leu Phe Ile Ser Cys Asn Asn Ser Gly Lys Gly Gly Asp
                   165                 170                 175
   Ser Ala Ser Thr Asn Pro Ala Asp Glu Ser Ala Lys Gly Pro Asn Leu
               180                 185                 190
   Thr Glu Ile Ser Lys Lys Ile Thr Asp Ser Asn Ala Phe Val Leu Ala
           195                 200                 205
   Val Lys Glu Val Glu Thr Leu Val Leu Ser Ile Asp Glu Leu Ala Lys
       210                 215                 220
   Lys Ala Ile Gly Gln Lys Ile Asp Asn Asn Asn Gly Leu Ala Ala Leu
   225                 230                 235                 240
   Asn Asn Gln Asn Gly Ser Leu Leu Ala Gly Ala Tyr Ala Ile Ser Thr
                   245                 250                 255
   Leu Ile Thr Glu Lys Leu Ser Lys Leu Lys Asn Leu Glu Glu Leu Lys
               260                 265                 270
   Thr Glu Ile Ala Lys Ala Lys Lys Cys Ser Glu Glu Phe Thr Asn Lys
           275                 280                 285
   Leu Lys Ser Gly His Ala Asp Leu Gly Lys Gln Asp Ala Thr Asp Asp
       290                 295                 300
   His Ala Lys Ala Ala Ile Leu Lys Thr His Ala Thr Thr Asp Lys Gly
   305                 310                 315                 320
   Ala Lys Glu Phe Lys Asp Leu Phe Glu Ser Val Glu Gly Leu Leu Lys
                   325                 330                 335
   Ala Ala Gln Val Ala Leu Thr Asn Ser Val Lys Glu Leu Thr Ser Pro
               340                 345                 350
   Val Val Ala Glu Ser Pro Lys Lys Pro
   355 360
<210> 33
   <211> 370
   <212> PRT
   <213> Borrelia sp.
<400> 33
   Met Arg Gly Ser His His His His His His Ser Leu Thr Gly Lys Ala
   1               5                   10                  15
   Arg Leu Glu Ser Ser Val Lys Asp Ile Thr Asn Glu Ile Glu Lys Ala
               20                  25                  30
   Ile Lys Glu Ala Glu Asp Ala Gly Val Lys Thr Asp Ala Phe Thr Glu
           35                  40                  45
   Thr Gln Thr Gly Gly Lys Val Ala Gly Pro Lys Ile Arg Ala Ala Lys
       50                  55                  60
   Ile Arg Val Ala Asp Leu Thr Ile Lys Phe Leu Glu Ala Thr Glu Glu
   65                  70                  75                  80
   Glu Thr Ile Thr Phe Lys Glu Asn Gly Ala Gly Glu Asp Glu Phe Ser
                   85                  90                  95
   Gly Ile Tyr Asp Leu Ile Leu Asn Ala Ala Lys Ala Val Glu Lys Ile
               100                 105                 110
   Gly Met Lys Asp Met Thr Lys Thr Val Glu Glu Ala Ala Lys Glu Asn
           115                 120                 125
   Pro Lys Thr Thr Ala Asn Gly Ile Ile Glu Ile Val Lys Val Met Lys
       130                 135                 140
   Ala Lys Val Glu Asn Ile Lys Glu Lys Gln Thr Lys Asn Gln Lys Lys
   145                 150                 155                 160
   Lys Asn Thr Leu Ser Ala Ile Leu Met Thr Leu Phe Leu Phe Ile Ser
                   165                 170                 175
   Cys Asn Asn Ser Gly Lys Gly Gly Asp Ser Ala Ser Thr Asn Pro Ala
               180                 185                 190
   Asp Glu Ser Ala Lys Gly Pro Asn Leu Thr Glu Ile Ser Lys Lys Ile
           195                 200                 205
   Thr Asp Ser Asn Ala Phe Val Leu Ala Val Lys Glu Val Glu Thr Leu
       210                 215                 220
   Val Leu Ser Ile Asp Glu Leu Ala Lys Lys Ala Ile Gly Gln Lys Ile
   225 230 235 240
   Asp Asn Asn Asn Gly Leu Ala Ala Leu Asn Asn Gln Asn Gly Ser Leu
   245 250 255
   Leu Ala Gly Ala Tyr Ala Ile Ser Thr Leu Ile Thr Glu Lys Leu Ser
   260 265 270
   Lys Leu Lys Asn Leu Glu Glu Leu Lys Thr Glu Ile Ala Lys Ala Lys
   275 280 285
   Lys Cys Ser Glu Glu Phe Thr Asn Lys Leu Lys Ser Gly His Ala Asp
   290 295 300
   Leu Gly Lys Gln Asp Ala Thr Asp Asp His Ala Lys Ala Ala Ile Leu
   305 310 315 320
   Lys Thr His Ala Thr Thr Asp Lys Gly Ala Lys Glu Phe Lys Asp Leu
   325 330 335
   Phe Glu Ser Val Glu Gly Leu Leu Lys Ala Ala Gln Val Ala Leu Thr
   340 345 350
   Asn Ser Val Lys Glu Leu Thr Ser Pro Val Val Ala Glu Ser Pro Lys
   355 360 365
   Lys Pro
   370
<210> 34
   <211> 350
   <212> PRT
   <213> Borrelia sp.
<400> 34
   Met Thr Gly Ala Thr Lys Ile Arg Leu Glu Arg Ser Ala Lys Asp Ile
   1 5 10 15
   Thr Asp Glu Ile Asp Ala Ile Lys Lys Asp Ala Ala Leu Lys Gly Val
   20 25 30
   Asn Phe Asp Ala Phe Lys Asp Lys Lys Thr Gly Ser Gly Val Ser Glu
   35 40 45
   Asn Pro Phe Ile Leu Glu Ala Lys Val Arg Ala Thr Thr Val Ala Glu
   50 55 60
   Lys Phe Val Ile Ala Ile Glu Glu Glu Ala Thr Lys Leu Lys Glu Thr
   65                  70                  75                  80
   Gly Ser Ser Gly Glu Phe Ser Ala Met Tyr Asp Leu Met Phe Glu Val
                   85                  90                  95
   Ser Lys Pro Leu Gln Lys Leu Gly Ile Gln Glu Met Thr Lys Thr Val
               100                 105                 110
   Ser Asp Ala Ala Glu Glu Asn Pro Pro Thr Thr Ala Gln Gly Val Leu
           115                 120                 125
   Glu Ile Ala Lys Lys Met Arg Glu Lys Leu Gln Arg Val His Thr Lys
       130                 135                 140
   Asn Tyr Cys Thr Leu Lys Lys Lys Glu Asn Ser Thr Phe Thr Asp Glu
   145                 150                 155                 160
   Lys Cys Lys Asn Asn Asn Thr Ser Ala Asn Ser Ala Asp Glu Ser Val
                   165                 170                 175
   Lys Gly Pro Asn Leu Thr Glu Ile Ser Lys Lys Ile Thr Asp Ser Asn
               180                 185                 190
   Ala Val Leu Leu Ala Val Lys Glu Val Glu Ala Leu Leu Ser Ser Ile
           195                 200                 205
   Asp Glu Ile Ala Ala Lys Ala Ile Gly Lys Lys Ile His Gln Asn Asn
       210                 215                 220
   Gly Leu Asp Thr Glu Asn Asn His Asn Gly Ser Leu Leu Ala Gly Ala
   225                 230                 235                 240
   Tyr Ala Ile Ser Thr Leu Ile Lys Gln Lys Leu Asp Gly Leu Lys Asn
                   245                 250                 255
   Glu Gly Leu Lys Glu Lys Ile Asp Ala Ala Lys Lys Cys Ser Glu Thr
               260                 265                 270
   Phe Thr Asn Lys Leu Lys Glu Lys His Thr Asp Ser Phe Gly Lys Glu
           275                 280                 285
   Gly Val Thr Asp Ala Asp Ala Lys Glu Ala Ile Leu Lys Thr Asn Gly
       290                 295                 300
   Thr Lys Thr Lys Gly Ala Glu Glu Leu Gly Lys Leu Phe Glu Ser Val
   305                 310                 315                 320
   Glu Val Leu Ser Lys Ala Ala Lys Glu Met Leu Ala Asn Ser Val Lys
   325 330 335
   Glu Leu Thr Ser Pro Val Val Ala Glu Ser Pro Lys Lys Pro
   340 345 350
<210> 35
   <211> 359
   <212> PRT
   <213> Borrelia sp.
<400> 35
   Met Arg Gly Ser His His His His His His Thr Gly Ala Thr Lys Ile
   1               5                   10                  15
   Arg Leu Glu Arg Ser Ala Lys Asp Ile Thr Asp Glu Ile Asp Ala Ile
               20                  25                  30
   Lys Lys Asp Ala Ala Leu Lys Gly Val Asn Phe Asp Ala Phe Lys Asp
           35                  40                  45
   Lys Lys Thr Gly Ser Gly Val Ser Glu Asn Pro Phe Ile Leu Glu Ala
       50                  55                  60
   Lys Val Arg Ala Thr Thr Val Ala Glu Lys Phe Val Ile Ala Ile Glu
   65                  70                  75                  80
   Glu Glu Ala Thr Lys Leu Lys Glu Thr Gly Ser Ser Gly Glu Phe Ser
                   85                  90                  95
   Ala Met Tyr Asp Leu Met Phe Glu Val Ser Lys Pro Leu Gln Lys Leu
               100                 105                 110
   Gly Ile Gln Glu Met Thr Lys Thr Val Ser Asp Ala Ala Glu Glu Asn
           115                 120                 125
   Pro Pro Thr Thr Ala Gln Gly Val Leu Glu Ile Ala Lys Lys Met Arg
       130                 135                 140
   Glu Lys Leu Gln Arg Val His Thr Lys Asn Tyr Cys Thr Leu Lys Lys
   145                 150                 155                 160
   Lys Glu Asn Ser Thr Phe Thr Asp Glu Lys Cys Lys Asn Asn Asn Thr
                   165                 170                 175
   Ser Ala Asn Ser Ala Asp Glu Ser Val Lys Gly Pro Asn Leu Thr Glu
               180                 185                 190
   Ile Ser Lys Lys Ile Thr Asp Ser Asn Ala Val Leu Leu Ala Val Lys
           195                 200                 205
   Glu Val Glu Ala Leu Leu Ser Ser Ile Asp Glu Ile Ala Ala Lys Ala
   210 215 220
   Ile Gly Lys Lys Ile His Gln Asn Asn Gly Leu Asp Thr Glu Asn Asn
   225 230 235 240
   His Asn Gly Ser Leu Leu Ala Gly Ala Tyr Ala Ile Ser Thr Leu Ile
   245 250 255
   Lys Gln Lys Leu Asp Gly Leu Lys Asn Glu Gly Leu Lys Glu Lys Ile
   260 265 270
   Asp Ala Ala Lys Lys Cys Ser Glu Thr Phe Thr Asn Lys Leu Lys Glu
   275 280 285
   Lys His Thr Asp Ser Phe Gly Lys Glu Gly Val Thr Asp Ala Asp Ala
   290 295 300
   Lys Glu Ala Ile Leu Lys Thr Asn Gly Thr Lys Thr Lys Gly Ala Glu
   305 310 315 320
   Glu Leu Gly Lys Leu Phe Glu Ser Val Glu Val Leu Ser Lys Ala Ala
   325 330 335
   Lys Glu Met Leu Ala Asn Ser Val Lys Glu Leu Thr Ser Pro Val Val
   340 345 350
   Ala Glu Ser Pro Lys Lys Pro
   355
<210> 36
   <211> 493
   <212> PRT
   <213> Borrelia sp.
<400> 36
   Met Thr Gly Glu Thr Lys Ile Arg Leu Glu Ser Ser Ala Gln Glu Ile
   1               5                   10                  15
   Lys Asp Glu Ile Asn Lys Ile Lys Ala Asn Ala Lys Lys Glu Gly Val
               20                  25                  30
   Lys Phe Glu Ala Phe Thr Asp Lys Gln Thr Gly Ser Lys Val Ser Glu
           35                  40                  45
   Lys Pro Glu Phe Ile Leu Lys Ala Lys Ile Lys Ala Ile Gln Val Ala
       50                  55                  60
   Glu Lys Phe Val Lys Ala Ile Lys Glu Glu Ala Glu Lys Leu Lys Lys
   65 70 75 80
   Ser Gly Ser Ser Gly Ala Phe Ser Ala Met Tyr Asp Leu Met Leu Asp
   85 90 95
   Val Ser Lys Pro Leu Glu Glu Ile Gly Ile Gln Lys Met Thr Gly Thr
   100 105 110
   Val Thr Lys Glu Ala Glu Lys Thr Pro Pro Thr Thr Ala Glu Gly Ile
   115 120 125
   Leu Ala Ile Ala Gln Ala Met Glu Glu Lys Leu Asn Asn Val Asn Lys
   130 135 140
   Lys Gln Gln Asp Ala Leu Lys Asn Leu Glu Glu Lys Ala Asn Thr Ala
   145 150 155 160
   Ala Thr Thr Ser Gly Thr Gly Lys Ala Arg Leu Glu Ser Ser Val Lys
   165 170 175
   Asp Ile Thr Asp Glu Ile Asp Lys Ala Ile Lys Glu Ala Ile Ala Asp
   180 185 190
   Gly Val Lys Leu Asn Glu Leu Glu Glu Asn Lys Thr Gly Ala Lys Lys
   195 200 205
   Gly Gly Pro Gln Ile Arg Asp Ala Lys Ile Arg Val Ile Asn Leu Ser
   210 215 220
   Val Lys Phe Leu Lys Glu Ile Glu Glu Glu Ala Asn Ile Leu Lys Asp
   225 230 235 240
   Asn Val Gly Met Asn Lys Val Asp Lys Asp Gln Leu Leu Lys Asp Met
   245 250 255
   Tyr Asp Leu Met Leu Asn Ala Ala Gly Ser Leu Gln Lys Leu Gly Leu
   260 265 270
   Gln Glu Met Ile Lys Thr Val Thr Gln Ala Ala Glu Lys Thr Pro Pro
   275 280 285
   Thr Thr Val Glu Gly Ile Leu Met Ile Ala Asn Thr Ile Glu Asp Lys
   290 295 300
   Leu Lys Lys Ile Lys Gly Lys Gln Glu Thr Asn Lys Lys Asp Glu Ser
   305 310 315 320
   Ala Lys Gly Pro Asn Leu Thr Val Ile Ser Lys Lys Ile Thr Asp Ser
   325 330 335
   Asn Ala Phe Leu Leu Ala Val Lys Glu Val Glu Ala Leu Leu Ser Ser
               340                 345                 350
   Ile Asp Glu Leu Ser Lys Ala Ile Gly Lys Lys Ile Lys Asn Asp Gly
           355                 360                 365
   Thr Leu Asp Asn Glu Ala Asn Arg Asn Glu Ser Leu Ile Ala Gly Ala
       370                 375                 380
   Tyr Glu Ile Ser Lys Leu Ile Thr Gln Lys Leu Ser Val Leu Asn Ser
   385                 390                 395                 400
   Glu Glu Leu Lys Glu Lys Ile Lys Glu Ala Lys Asp Cys Ser Glu Lys
                   405                 410                 415
   Phe Thr Thr Lys Leu Lys Asp Ser His Ala Glu Leu Gly Ile Gln Ser
               420                 425                 430
   Val Gln Asp Asp Asn Ala Lys Lys Ala Ile Leu Lys Thr His Gly Thr
           435                 440                 445
   Lys Asp Lys Gly Ala Lys Glu Leu Glu Glu Leu Phe Lys Ser Leu Glu
       450                 455                 460
   Ser Leu Ser Lys Ala Ala Gln Ala Ala Leu Thr Asn Ser Val Lys Glu
   465                 470                 475                 480
   Leu Thr Asn Pro Val Val Ala Glu Ser Pro Lys Lys Pro
                   485                 490
<210> 37
   <211> 502
   <212> PRT
   <213> Borrelia sp.
<400> 37
   Met Arg Gly Ser His His His His His His Thr Gly Glu Thr Lys Ile
   1               5                   10                  15
   Arg Leu Glu Ser Ser Ala Gln Glu Ile Lys Asp Glu Ile Asn Lys Ile
               20                  25                  30
   Lys Ala Asn Ala Lys Lys Glu Gly Val Lys Phe Glu Ala Phe Thr Asp
           35                  40                  45
   Lys Gln Thr Gly Ser Lys Val Ser Glu Lys Pro Glu Phe Ile Leu Lys
       50                  55                  60
   Ala Lys Ile Lys Ala Ile Gln Val Ala Glu Lys Phe Val Lys Ala Ile
   65 70 75 80
   Lys Glu Glu Ala Glu Lys Leu Lys Lys Ser Gly Ser Ser Gly Ala Phe
   85 90 95
   Ser Ala Met Tyr Asp Leu Met Leu Asp Val Ser Lys Pro Leu Glu Glu
   100 105 110
   Ile Gly Ile Gln Lys Met Thr Gly Thr Val Thr Lys Glu Ala Glu Lys
   115 120 125
   Thr Pro Pro Thr Thr Ala Glu Gly Ile Leu Ala Ile Ala Gln Ala Met
   130 135 140
   Glu Glu Lys Leu Asn Asn Val Asn Lys Lys Gln Gln Asp Ala Leu Lys
   145 150 155 160
   Asn Leu Glu Glu Lys Ala Asn Thr Ala Ala Thr Thr Ser Gly Thr Gly
   165 170 175
   Lys Ala Arg Leu Glu Ser Ser Val Lys Asp Ile Thr Asp Glu Ile Asp
   180 185 190
   Lys Ala Ile Lys Glu Ala Ile Ala Asp Gly Val Lys Leu Asn Glu Leu
   195 200 205
   Glu Glu Asn Lys Thr Gly Ala Lys Lys Gly Gly Pro Gln Ile Arg Asp
   210 215 220
   Ala Lys Ile Arg Val Ile Asn Leu Ser Val Lys Phe Leu Lys Glu Ile
   225 230 235 240
   Glu Glu Glu Ala Asn Ile Leu Lys Asp Asn Val Gly Met Asn Lys Val
   245 250 255
   Asp Lys Asp Gln Leu Leu Lys Asp Met Tyr Asp Leu Met Leu Asn Ala
   260 265 270
   Ala Gly Ser Leu Gln Lys Leu Gly Leu Gln Glu Met Ile Lys Thr Val
   275 280 285
   Thr Gln Ala Ala Glu Lys Thr Pro Pro Thr Thr Val Glu Gly Ile Leu
   290 295 300
   Met Ile Ala Asn Thr Ile Glu Asp Lys Leu Lys Lys Ile Lys Gly Lys
   305 310 315 320
   Gln Glu Thr Asn Lys Lys Asp Glu Ser Ala Lys Gly Pro Asn Leu Thr
   325 330 335
   Val Ile Ser Lys Lys Ile Thr Asp Ser Asn Ala Phe Leu Leu Ala Val
   340 345 350
   Lys Glu Val Glu Ala Leu Leu Ser Ser Ile Asp Glu Leu Ser Lys Ala
   355 360 365
   Ile Gly Lys Lys Ile Lys Asn Asp Gly Thr Leu Asp Asn Glu Ala Asn
   370 375 380
   Arg Asn Glu Ser Leu Ile Ala Gly Ala Tyr Glu Ile Ser Lys Leu Ile
   385 390 395 400
   Thr Gln Lys Leu Ser Val Leu Asn Ser Glu Glu Leu Lys Glu Lys Ile
   405 410 415
   Lys Glu Ala Lys Asp Cys Ser Glu Lys Phe Thr Thr Lys Leu Lys Asp
   420 425 430
   Ser His Ala Glu Leu Gly Ile Gln Ser Val Gln Asp Asp Asn Ala Lys
   435 440 445
   Lys Ala Ile Leu Lys Thr His Gly Thr Lys Asp Lys Gly Ala Lys Glu
   450 455 460
   Leu Glu Glu Leu Phe Lys Ser Leu Glu Ser Leu Ser Lys Ala Ala Gln
   465 470 475 480
   Ala Ala Leu Thr Asn Ser Val Lys Glu Leu Thr Asn Pro Val Val Ala
   485 490 495
   Glu Ser Pro Lys Lys Pro
   500
<210> 38
   <211> 506
   <212> PRT
   <213> Borrelia sp.
<400> 38
   Met Arg Gly Ser His His His His His His Thr Gly Glu Thr Lys Ile
   1               5                   10                  15
   Arg Leu Glu Ser Ser Ala Gln Glu Ile Lys Asp Glu Ile Asn Lys Ile
               20                  25                  30
   Lys Ala Asn Ala Lys Lys Glu Gly Val Lys Phe Glu Ala Phe Thr Asp
           35                  40                  45
   Lys Gln Thr Gly Ser Lys Val Ser Glu Lys Pro Glu Phe Ile Leu Lys
       50                  55                  60
   Ala Lys Ile Lys Ala Ile Gln Val Ala Glu Lys Phe Val Lys Ala Ile
   65                  70                  75                  80
   Lys Glu Glu Ala Glu Lys Leu Lys Lys Ser Gly Ser Ser Gly Ala Phe
                   85                  90                  95
   Ser Ala Met Tyr Asp Leu Met Leu Asp Val Ser Lys Pro Leu Glu Glu
               100                 105                 110
   Ile Gly Ile Gln Lys Met Thr Gly Thr Val Thr Lys Glu Ala Glu Lys
           115                 120                 125
   Thr Pro Pro Thr Thr Ala Glu Gly Ile Leu Ala Ile Ala Gln Ala Met
       130                 135                 140
   Glu Glu Lys Leu Asn Asn Val Asn Lys Lys Gln Gln Asp Ala Leu Lys
   145                 150                 155                 160
   Asn Leu Glu Glu Lys Ala Asn Thr Ala Ala Thr Thr Ser Gly Thr Gly
                   165                 170                 175
   Lys Ala Arg Leu Glu Ser Ser Val Lys Asp Ile Thr Asp Glu Ile Asp
               180                 185                 190
   Lys Ala Ile Lys Glu Ala Ile Ala Asp Gly Val Lys Leu Asn Glu Leu
           195                 200                 205
   Glu Glu Asn Lys Thr Gly Ala Lys Lys Gly Gly Pro Gln Ile Arg Asp
       210                 215                 220
   Ala Lys Ile Arg Val Ile Asn Leu Ser Val Lys Phe Leu Lys Glu Ile
   225                 230                 235                 240
   Glu Glu Glu Ala Asn Ile Leu Lys Asp Asn Val Gly Met Asn Lys Val
                   245                 250                 255
   Asp Lys Asp Gln Leu Leu Lys Asp Met Tyr Asp Leu Met Leu Asn Ala
               260                 265                 270
   Ala Gly Ser Leu Gln Lys Leu Gly Leu Gln Glu Met Ile Lys Thr Val
           275                 280                 285
   Thr Gln Ala Ala Glu Lys Thr Pro Pro Thr Thr Val Glu Gly Ile Leu
       290                 295                 300
   Met Ile Ala Asn Thr Ile Glu Asp Lys Leu Lys Lys Ile Lys Gly Lys
   305                 310                 315                 320
   Gln Glu Thr Asn Lys Lys Gly Ser Gly Gly Asp Glu Ser Ala Lys Gly
                   325                 330                 335
   Pro Asn Leu Thr Val Ile Ser Lys Lys Ile Thr Asp Ser Asn Ala Phe
               340                 345                 350
   Leu Leu Ala Val Lys Glu Val Glu Ala Leu Leu Ser Ser Ile Asp Glu
           355                 360                 365
   Leu Ser Lys Ala Ile Gly Lys Lys Ile Lys Asn Asp Gly Thr Leu Asp
       370                 375                 380
   Asn Glu Ala Asn Arg Asn Glu Ser Leu Ile Ala Gly Ala Tyr Glu Ile
   385                 390                 395                 400
   Ser Lys Leu Ile Thr Gln Lys Leu Ser Val Leu Asn Ser Glu Glu Leu
                   405                 410                 415
   Lys Glu Lys Ile Lys Glu Ala Lys Asp Cys Ser Glu Lys Phe Thr Thr
               420                 425                 430
   Lys Leu Lys Asp Ser His Ala Glu Leu Gly Ile Gln Ser Val Gln Asp
           435                 440                 445
   Asp Asn Ala Lys Lys Ala Ile Leu Lys Thr His Gly Thr Lys Asp Lys
       450                 455                 460
   Gly Ala Lys Glu Leu Glu Glu Leu Phe Lys Ser Leu Glu Ser Leu Ser
   465                 470                 475                 480
   Lys Ala Ala Gln Ala Ala Leu Thr Asn Ser Val Lys Glu Leu Thr Asn
                   485                 490                 495
   Pro Val Val Ala Glu Ser Pro Lys Lys Pro
               500                 505
<210> 39
   <211> 1086
   <212> DNA
   <213> Borrelia sp.
<400> 39
   atgagcctga ccggcaaagc gcgtctggaa agcagcgtga aagatattac caacgaaatt 60
   gaaaaagcga ttaaagaagc ggaagatgcg ggcgtgaaaa ccgatgcgtt taccgaaacc 120
   cagaccggcg gcaaagtggc gggcccgaaa attcgtgcgg cgaaaattcg tgtggcggat 180
   ctgaccatta aatttctgga agcgaccgaa gaagaaacca ttacctttaa agaaaatggc 240
   gcgggcgaag atgaatttag cggcatttat gatctgattc tgaacgcggc gaaagcggtg 300
   gaaaaaattg gcatgaaaga tatgaccaaa accgtggaag aagcggcgaa agaaaatccg 360
   aaaaccaccg cgaacggtat tattgaaatt gtgaaagtga tgaaagcgaa agtggaaaat 420
   attaaagaaa aacagaccaa aaaccagaaa aaaaaaaaca ccctgagcgc gattctgatg 480
   accctgtttc tgtttattag ctgcaacaac agcggcaaag gcggcgatag cgcgagcacc 540
   aacccggcgg atgaaagcgc gaaaggcccg aacctgaccg aaattagcaa aaaaatcacc 600
   gatagcaacg cgtttgtgct ggcggtgaaa gaagtggaaa ccctggttct gagcattgat 660
   gaactggcga aaaaagcgat tggccagaaa atcgataaca acaacggcct ggcggcgctg 720
   aacaaccaga acggcagcct gctggcgggt gcgtatgcga ttagcaccct gattaccgaa 780
   aaactgagca aactgaaaaa cctggaagaa ctgaaaaccg aaatcgcgaa agcgaaaaaa 840
   tgcagcgaag aatttaccaa caaactgaaa agcggccatg cggatctggg caaacaggat 900
   gcgaccgatg atcatgcgaa agcggcgatt ctgaaaaccc atgcgaccac cgataaaggc 960
   gcgaaagaat ttaaagacct gttcgaaagc gtggaaggcc tgctgaaagc ggcgcaggtg 1020
   gcgctgacca acagcgtgaa agaactgacc agcccggtgg ttgcggaaag cccgaaaaaa 1080
   ccgtaa 1086
<210> 40
   <211> 1113
   <212> DNA
   <213> Borrelia sp.
<400> 40
   atgaggggat cccatcatca tcatcatcat agcctgaccg gcaaagcgcg tctggaaagc      60
   agcgtgaaag atattaccaa cgaaattgaa aaagcgatta aagaagcgga agatgcgggc     120
   gtgaaaaccg atgcgtttac cgaaacccag accggcggca aagtggcggg cccgaaaatt     180
   cgtgcggcga aaattcgtgt ggcggatctg accattaaat ttctggaagc gaccgaagaa     240
   gaaaccatta cctttaaaga aaatggcgcg ggcgaagatg aatttagcgg catttatgat     300
   ctgattctga acgcggcgaa agcggtggaa aaaattggca tgaaagatat gaccaaaacc     360
   gtggaagaag cggcgaaaga aaatccgaaa accaccgcga acggtattat tgaaattgtg     420
   aaagtgatga aagcgaaagt ggaaaatatt aaagaaaaac agaccaaaaa ccagaaaaaa     480
   aaaaacaccc tgagcgcgat tctgatgacc ctgtttctgt ttattagctg caacaacagc     540
   ggcaaaggcg gcgatagcgc gagcaccaac ccggcggatg aaagcgcgaa aggcccgaac     600
   ctgaccgaaa ttagcaaaaa aatcaccgat agcaacgcgt ttgtgctggc ggtgaaagaa     660
   gtggaaaccc tggttctgag cattgatgaa ctggcgaaaa aagcgattgg ccagaaaatc     720
   gataacaaca acggcctggc ggcgctgaac aaccagaacg gcagcctgct ggcgggtgcg     780
   tatgcgatta gcaccctgat taccgaaaaa ctgagcaaac tgaaaaacct ggaagaactg     840
   aaaaccgaaa tcgcgaaagc gaaaaaatgc agcgaagaat ttaccaacaa actgaaaagc     900
   ggccatgcgg atctgggcaa acaggatgcg accgatgatc atgcgaaagc ggcgattctg     960
   aaaacccatg cgaccaccga taaaggcgcg aaagaattta aagacctgtt cgaaagcgtg    1020
   gaaggcctgc tgaaagcggc gcaggtggcg ctgaccaaca gcgtgaaaga actgaccagc    1080
   ccggtggttg cggaaagccc gaaaaaaccg taa                                 1113
<210> 41
   <211> 1053
   <212> DNA
   <213> Borrelia sp.
<400> 41
   atgaccggcg cgaccaaaat ccgcctggaa cgcagcgcga aagatatcac agatgaaatc      60
   gatgcgatca agaaagacgc ggcgctgaaa ggcgtcaact ttgatgcatt taaagataaa     120
   aagaccgggt ctggagttag cgagaatcca tttattctgg aagcgaaagt tcgtgctacg     180
   acggtggcag aaaaatttgt gattgcgatt gaagaagaag caacgaaact gaaagaaacc     240
   ggcagcagtg gcgaatttag tgcgatgtat gacctgatgt ttgaggtctc taaaccgctg     300
   cagaaactgg ggattcaaga aatgaccaag acggtatctg atgcagcgga agaaaacccg     360
   cctacgacgg cgcaaggcgt cctggaaatt gccaagaaaa tgcgcgaaaa actgcaacgc     420
   gttcatacca aaaattattg cactctgaag aagaaagaga atagcacttt tacggatgaa     480
   aaatgtaaaa ataataacac cagcgcgaac agcgcggatg aaagcgtgaa aggcccgaac     540
   ctgaccgaaa ttagcaaaaa aatcaccgat agcaacgcgg tgctgctggc ggtgaaagaa     600
   gtggaagcgc tgctgagcag cattgatgaa attgcggcga aagcgattgg caaaaaaatc     660
   catcagaaca acggcctgga taccgaaaac aaccataacg gcagcctgct ggcgggtgcg     720
   tatgcgatta gcaccctgat taaacagaaa ctggatggcc tgaaaaacga aggcttaaaa     780
   gaaaaaattg atgcggcgaa aaaatgcagc gaaaccttca ccaacaaact gaaagaaaaa     840
   cataccgata gcttcggtaa agaaggcgtg accgacgcgg atgcgaaaga agcgattctg     900
   aaaaccaacg gcaccaaaac caaaggcgcg gaagaactgg gcaaactgtt tgaaagcgtg     960
   gaagttctga gcaaagcggc caaagaaatg ctggcgaaca gcgtgaaaga actgaccagc    1020
   ccggtggtgg cagaatctcc gaaaaagccc taa                                1053
<210> 42
   <211> 1080
   <212> DNA
   <213> Borrelia sp.
<400> 42
   atgaggggat cccatcatca tcatcatcac accggcgcga ccaaaatccg cctggaacgc      60
   agcgcgaaag atatcacaga tgaaatcgat gcgatcaaga aagacgcggc gctgaaaggc     120
   gtcaactttg atgcatttaa agataaaaag accgggtctg gagttagcga gaatccattt     180
   attctggaag cgaaagttcg tgctacgacg gtggcagaaa aatttgtgat tgcgattgaa     240
   gaagaagcaa cgaaactgaa agaaaccggc agcagtggcg aatttagtgc gatgtatgac     300
   ctgatgtttg aggtctctaa accgctgcag aaactgggga ttcaagaaat gaccaagacg     360
   gtatctgatg cagcggaaga aaacccgcct acgacggcgc aaggcgtcct ggaaattgcc     420
   aagaaaatgc gcgaaaaact gcaacgcgtt cataccaaaa attattgcac tctgaagaag     480
   aaagagaata gcacttttac ggatgaaaaa tgtaaaaata ataacaccag cgcgaacagc     540
   gcggatgaaa gcgtgaaagg cccgaacctg accgaaatta gcaaaaaaat caccgatagc     600
   aacgcggtgc tgctggcggt gaaagaagtg gaagcgctgc tgagcagcat tgatgaaatt     660
   gcggcgaaag cgattggcaa aaaaatccat cagaacaacg gcctggatac cgaaaacaac     720
   cataacggca gcctgctggc gggtgcgtat gcgattagca ccctgattaa acagaaactg     780
   gatggcctga aaaacgaagg cttaaaagaa aaaattgatg cggcgaaaaa atgcagcgaa     840
   accttcacca acaaactgaa agaaaaacat accgatagct tcggtaaaga aggcgtgacc     900
   gacgcggatg cgaaagaagc gattctgaaa accaacggca ccaaaaccaa aggcgcggaa     960
   gaactgggca aactgtttga aagcgtggaa gttctgagca aagcggccaa agaaatgctg    1020
   gcgaacagcg tgaaagaact gaccagcccg gtggtggcag aatctccgaa aaagccctaa    1080
<210> 43
   <211> 1482
   <212> DNA
   <213> Borrelia sp.
<400> 43
   atgactggtg aaacgaaaat tcgtctggaa tcatccgctc aggagattaa agacgaaatc 60
   aacaaaatta aagcaaacgc caagaaagaa ggcgtgaagt ttgaagcgtt taccgataaa 120
   cagaccggca gcaaagtttc agaaaaaccg gagtttattc tgaaagccaa aattaaagcg 180
   atccaggttg cggaaaaatt cgtgaaagcg attaaagaag aagccgaaaa actgaaaaaa 240
   tctggttcga gcggcgcatt ttccgcaatg tatgatctga tgctggatgt aagcaaaccg 300
   ctggaagaga ttggcattca gaaaatgacc ggcactgtca caaaggaagc ggaaaaaaca 360
   ccgccaacca ctgcagaagg gattctggcg atcgcccagg cgatggaaga gaaactgaac 420
   aacgttaata aaaaacagca ggatgcactg aaaaacctgg aagagaaggc gaacaccgcg 480
   gcgactacgt cagggaccgg taaagcgcgt ctggaaagct cggtaaaaga tatcacagac 540
   gaaattgaca aagccatcaa agaagccatt gcagacggcg ttaaactgaa tgaactggaa 600
   gaaaataaaa ccggtgcgaa aaaaggtggc ccgcagattc gcgatgcgaa aatccgtgtg 660
   atcaacctga gcgttaaatt cctgaaagaa atcgaggagg aagcaaacat cctgaaggat 720
   aatgttggca tgaacaaggt agataaagat cagctgctga aagacatgta cgacctgatg 780
   ctgaacgcgg ctggcagtct gcagaaactg ggtctgcagg aaatgatcaa aacggttacc 840
   caagctgcgg aaaaaacccc accgaccacg gttgaaggca ttctgatgat tgcaaacacc 900
   attgaagaca aactgaagaa aatcaaaggc aaacaggaaa caaacaaaaa agatgaaagc 960
   gcaaaaggcc cgaatctgac cgtcatttct aagaaaatta ccgattcaaa cgcgtttctg 1020
   ctggccgtga aagaggttga agccctgctg tcctcgattg atgaactgag caaagctatc 1080
   ggaaagaaaa ttaaaaatga tgggacgctg gataacgagg caaatcgcaa tgaaagcctg 1140
   attgcaggcg catatgaaat cagtaaactg attacacaga aactgagtgt cctgaacagc 1200
   gaagaactga aagaaaaaat caaagaagcc aaagactgtt cggaaaagtt tactaccaaa 1260
   ctgaaagact cgcatgctga actgggtatt cagtcagtgc aagatgataa tgcgaaaaaa 1320
   gcaattctga aaacgcacgg gacgaaagat aaaggtgcca aagagctgga agaactgttt 1380
   aaaagcctgg aatcgctgag taaagccgca caggccgcgc tgaccaatag cgtgaaggaa 1440
   ctgactaatc cggttgtagc agaatctccg aaaaagccgt aa 1482
<210> 44
   <211> 1509
   <212> DNA
   <213> Borrelia sp.
<400> 44
   atgaggggat cccatcatca ccaccatcat actggtgaaa cgaaaattcg tctggaatca 60
   tccgctcagg agattaaaga cgaaatcaac aaaattaaag caaacgccaa gaaagaaggc 120
   gtgaagtttg aagcgtttac cgataaacag accggcagca aagtttcaga aaaaccggag 180
   tttattctga aagccaaaat taaagcgatc caggttgcgg aaaaattcgt gaaagcgatt 240
   aaagaagaag ccgaaaaact gaaaaaatct ggttcgagcg gcgcattttc cgcaatgtat 300
   gatctgatgc tggatgtaag caaaccgctg gaagagattg gcattcagaa aatgaccggc 360
   actgtcacaa aggaagcgga aaaaacaccg ccaaccactg cagaagggat tctggcgatc 420
   gcccaggcga tggaagagaa actgaacaac gttaataaaa aacagcagga tgcactgaaa 480
   aacctggaag agaaggcgaa caccgcggcg actacgtcag ggaccggtaa agcgcgtctg 540
   gaaagctcgg taaaagatat cacagacgaa attgacaaag ccatcaaaga agccattgca 600
   gacggcgtta aactgaatga actggaagaa aataaaaccg gtgcgaaaaa aggtggcccg 660
   cagattcgcg atgcgaaaat ccgtgtgatc aacctgagcg ttaaattcct gaaagaaatc 720
   gaggaggaag caaacatcct gaaggataat gttggcatga acaaggtaga taaagatcag 780
   ctgctgaaag acatgtacga cctgatgctg aacgcggctg gcagtctgca gaaactgggt 840
   ctgcaggaaa tgatcaaaac ggttacccaa gctgcggaaa aaaccccacc gaccacggtt 900
   gaaggcattc tgatgattgc aaacaccatt gaagacaaac tgaagaaaat caaaggcaaa 960
   caggaaacaa acaaaaaaga tgaaagcgca aaaggcccga atctgaccgt catttctaag 1020
   aaaattaccg attcaaacgc gtttctgctg gccgtgaaag aggttgaagc cctgctgtcc 1080
   tcgattgatg aactgagcaa agctatcgga aagaaaatta aaaatgatgg gacgctggat 1140
   aacgaggcaa atcgcaatga aagcctgatt gcaggcgcat atgaaatcag taaactgatt 1200
   acacagaaac tgagtgtcct gaacagcgaa gaactgaaag aaaaaatcaa agaagccaaa 1260
   gactgttcgg aaaagtttac taccaaactg aaagactcgc atgctgaact gggtattcag 1320
   tcagtgcaag atgataatgc gaaaaaagca attctgaaaa cgcacgggac gaaagataaa 1380
   ggtgccaaag agctggaaga actgtttaaa agcctggaat cgctgagtaa agccgcacag 1440
   gccgcgctga ccaatagcgt gaaggaactg actaatccgg ttgtagcaga atctccgaaa 1500
   aagccgtaa 1509
<210> 45
   <211> 1521
   <212> DNA
   <213> Borrelia sp.
<400> 45
   atgaggggat cccatcatca ccaccatcat actggtgaaa cgaaaattcg tctggaatca 60
   tccgctcagg agattaaaga cgaaatcaac aaaattaaag caaacgccaa gaaagaaggc 120
   gtgaagtttg aagcgtttac cgataaacag accggcagca aagtttcaga aaaaccggag 180
   tttattctga aagccaaaat taaagcgatc caggttgcgg aaaaattcgt gaaagcgatt 240
   aaagaagaag ccgaaaaact gaaaaaatct ggttcgagcg gcgcattttc cgcaatgtat 300
   gatctgatgc tggatgtaag caaaccgctg gaagagattg gcattcagaa aatgaccggc 360
   actgtcacaa aggaagcgga aaaaacaccg ccaaccactg cagaagggat tctggcgatc 420
   gcccaggcga tggaagagaa actgaacaac gttaataaaa aacagcagga tgcactgaaa 480
   aacctggaag agaaggcgaa caccgcggcg actacgtcag ggaccggtaa agcgcgtctg 540
   gaaagctcgg taaaagatat cacagacgaa attgacaaag ccatcaaaga agccattgca 600
   gacggcgtta aactgaatga actggaagaa aataaaaccg gtgcgaaaaa aggtggcccg 660
   cagattcgcg atgcgaaaat ccgtgtgatc aacctgagcg ttaaattcct gaaagaaatc 720
   gaggaggaag caaacatcct gaaggataat gttggcatga acaaggtaga taaagatcag 780
   ctgctgaaag acatgtacga cctgatgctg aacgcggctg gcagtctgca gaaactgggt 840
   ctgcaggaaa tgatcaaaac ggttacccaa gctgcggaaa aaaccccacc gaccacggtt 900
   gaaggcattc tgatgattgc aaacaccatt gaagacaaac tgaagaaaat caaaggcaaa 960
   caggaaacaa acaaaaaagg ttccgggggt gatgaaagcg caaaaggccc gaatctgacc 1020
   gtcatttcta agaaaattac cgattcaaac gcgtttctgc tggccgtgaa agaggttgaa 1080
   gccctgctgt cctcgattga tgaactgagc aaagctatcg gaaagaaaat taaaaatgat 1140
   gggacgctgg ataacgaggc aaatcgcaat gaaagcctga ttgcaggcgc atatgaaatc 1200
   agtaaactga ttacacagaa actgagtgtc ctgaacagcg aagaactgaa agaaaaaatc 1260
   aaagaagcca aagactgttc ggaaaagttt actaccaaac tgaaagactc gcatgctgaa 1320
   ctgggtattc agtcagtgca agatgataat gcgaaaaaag caattctgaa aacgcacggg 1380
   acgaaagata aaggtgccaa agagctggaa gaactgttta aaagcctgga atcgctgagt 1440
   aaagccgcac aggccgcgct gaccaatagc gtgaaggaac tgactaatcc ggttgtagca 1500
   gaatctccga aaaagccgta a 1521
<210> 46
   <211> 4
   <212> PRT
   <213> Artificial
<220>
   <223> aa+ 2
<400> 46
   Gly Ser Gly Gly
   1
<210> 47
   <211> 12
   <212> DNA
   <213> Artificial
<220>
   <223> DNA aa+ 2
<400> 47
   ggttccgggg gt 12

## Description

La Borréliose de Lyme (LB) est une maladie infectieuse non contagieuse, due à un spirochète appelé *Borrelia burgdorferi,* transmis à l'homme par une piqûre de tique du genre *Ixodes.* La LB entraîne, sans traitement, des troubles pathologiques divers (dermatologiques, arthritiques, cardiaques, neurologiques et parfois oculaires). C'est la maladie à vecteur la plus fréquente aux USA et dans certains pays tempérés de l'hémisphère Nord.

Plusieurs espèces de borrélies, actuellement désignées sous le terme de groupe burgdorferi ou *Borrelia burgdorferi* sensu lato (incluant *Borrelia burgdorferi* sensu stricto, *B.garinii* et *B. afzelii*) sont impliquées dans cette infection. Ces espèces sont pathogènes pour l'homme.

Aux Etats-Unis, l'espèce infectieuse impliquée est *Borrelia burgdorferi* sensu stricto. En Europe, à cette espèce, s'ajoutent *B.garinii* et *B. afzelii*. En Asie, les espèces impliquées sont *B.garinii* et *B. afzelii.*

Aux Etats-Unis, environ 10.000 cas sont déclarés. En Europe les taux d'incidence varient de moins de 5 pour 100.000.

La Borreliose de Lyme évolue en passant par trois phases distinctes, de l'infection précoce à la phase tardive. Le stade précoce (Stade I), peut être asymptomatique ou se traduire par un tableau pseudo-grippal. Dans 50-80% des cas, on note l'apparition d'un rash cutané inflammatoire plusieurs jours après la morsure de la tique, d'aspect très particulier nommé érythème migrant (EM). En l'absence de traitement, la dissémination des Borrelia par voie sanguine se traduit quelques semaines plus tard par la survenue d'arthrites inflammatoires, d'atteintes neurologiques (neuroborréliose) et méningées, de manifestations cutanées et cardiaques (Stade II). Après plusieurs mois ou années, la maladie évolue vers une forme chronique atrophiante, encéphalopathie, encéphalomyélite et arthrite chronique (Stade III).

Il existe un tropisme organique particulier à chacune des espèces de *Borrelia burgdorferi.* Si le premier stade d'érythème migrant est indistinctement lié aux trois espèces, l'évolution vers une forme neurologique est préférentiellement associées à l'espèce *B. garinii,* les arthrites plutôt à B. *burgdorferi* sensu stricto, l'acrodermatite chronique atrophiante est spécifique de *B. afzelii.*

La ressemblance des symptômes cliniques entre la borréliose de Lyme et d'autres maladies non liées ainsi que la variabilité des manifestations rend le diagnostic clinique difficile. Le diagnostic de la borréliose peut être particulièrement difficile sur la base d'observations cliniques, si les preuves d'anamnèse sont absentes (morsure de tique ou EM). Le stade précoce de la maladie peut être inapparent jusqu'au moment ou elle atteint des stades cliniques très avancés.

C'est pourquoi le diagnostic de LB est basé sur des signes cliniques mais aussi sur la détection des anticorps spécifiques des *borrelia burgdorferi* pathogènes dans le sérum, le plus souvent par ELISA (Enzyme Linked ImmunoSorbent Assay) ou encore EIA, IFA.

En Europe, l'évaluation de la réponse sérologique est compliquée du fait de l'existence de trois espèces pathogènes et de la variabilité inter-espèces pour les antigènes immunodominants majeurs. Les antigènes actuellement utilisés en routine pour la détection des IgGs et IgMs de LB sont des échantillons cellulaires traités aux ultra sons de *Borrelia burgdorferi* sensu lato. Les performances des essais sérologiques avec ces antigènes en terme de spécificité et de sensibilité sont grandement variables. Ainsi, en raison d'une spécificité insuffisante, impliquant des réactivités croisées avec des anticorps associés à des bactéries pathogènes, notamment Treponema pallidum (agent étiologique de la syphilis), des spirochètes, *rickettisies, erlichia*, ou *Helicobacter pylori*, le diagnostic d'échantillons testés positifs en ELISA doit être confirmé par immunoblot. La sensibilité est également un facteur majeur. En effet, *Borrelia burgdorferi* sensu lato exprime différentes protéines de surface par adaptation à divers micro-environnements, de sorte que la diversité génétique et l'expression différentielle des gènes de *Borrelia burgdorferi* chez les patients ont des implications importantes pour le développement de tests sérologiques de LB.

Il était donc nécessaire de développer un kit qui pallie les inconvénients précités et qui plus particulièrement réponde aux critères de spécificité et de sensibilité attendus.

La protéine VlsE (surface expressed lipoprotein with Extensive antigenic Variation) est principalement exprimée in vivo, de façon transitoire et rapidement après infection de l'hôte. Elle est très immunogénique chez l'hôte infecté impliquant la production d'IgGs et d'IgMs. Le locus Vls est localisé sur un plasmide linéaire de 28 kb (Ip28-1) présent chez les trois génoespèces de borrelia responsables de Lyme et composé de cassettes silencieuses et d'un site d'expression (VlsE). *In vivo* des recombinaisons aléatoires entre cassettes d'expression et cassettes silencieuses surviennent au cours de l'infection et sont à l'origine de la variabilité antigénique de VlsE. La protéine VlsE est composée de six régions variables VR1-VR6, situées en surface de la protéine VlsE, espacées de régions invariables IR1-IR6.

Il est connu que les protéines VlsE présentent une importante hétérogénéité inter et intra-espèces. En 2004, Gôttner et al. [1] ont décrit une identité d'environ 47 à 58 au niveau protéique de VlsE provenant de quatre souches.

US2009/0162875 décrit des protéines chimériques comprenant la région IR6 de VIsE pour le diagnostic d'une Borréliose.

Pour pallier les problèmes de sensibilité et de spécificité précités, les inventeurs ont produit une protéine chimérique de Borrelia comprenant au moins une séquence du domaine extracellulaire d'une protéine VlsE d'une première espèce de Borrelia correspondant à une souche déterminée et au moins une séquence d'une région IR6 d'une protéine VlsE d'une seconde espèce de Borrelia ou de la première espèce de Borrelia mais correspondant à une souche différente de celle de la première espèce, ladite protéine chimérique comprenant (ou consistant essentiellement en ou encore consistant en):
- la séquence du domaine extracellulaire de la protéine VlsE de la première espèce de Borrelia qui est composée de cinq régions variables VR1, VR2, VR3, VR4 et VR5 et de six régions invariables IR1, IR2, IR3, IR4, IR5 et IR6, ladite au moins une séquence du domaine extracellulaire étant choisie dans le groupe consistant en SEQ ID NOs : 1, 2, 3, 4 et 5 ou un variant d'une desdites séquences SEQ ID NOs 1, 2, 3, 4 et 5, ledit variant présentant au moins 50% d'identité (de préférence au moins 60% ou au moins 70% d'identité et avantageusement au moins 80% ou au moins 85% d'identité) avec SEQ ID NOs 1, 2, 3, 4 et 5, respectivement, à la condition que ledit variant soit capable de former un complexe immunologique avec des anticorps produits suite à une infection par Borrelia, et
- la au moins une séquence de la région IR6 de la seconde espèce de Borrelia ou de la première espèce de Borrelia mais correspondant à une souche différente de celle de la première espèce étant choisie dans le groupe consistant en SEQ ID NOs : 6, 7 et 8 ou un variant d'une desdites séquences SEQ ID NOs 6, 7, et 8, ledit variant présentant au moins 80% d'identité (de préférence au moins 85% et avantageusement au moins 90% d'identité) avec SEQ ID NOs 6, 7, et 8, respectivement, à la condition que le variant de ladite séquence soit capable de former un complexe immunologique avec des anticorps produits suite à une infection par Borrelia.

La protéine chimérique identifiée ci-dessus peut de plus comprendre une séquence variable VR6 d'une espèce de Borrelia, cette séquence étant identifiée en SEQ ID NO : 9 dans le listage de séquences.

Une protéine chimère préférée comprend (ou consiste essentiellement en ou consiste en) :
- la séquence SEQ ID NO : 1 ou un variant de ladite séquence SEQ ID NO : 1, ledit variant présentant au moins 50% d'identité (de préférence au moins au moins 60% ou au moins 70% d'identité et avantageusement au moins 80% ou au moins 85% d'identité) avec SEQ ID NO : 1,
- la séquence SEQ ID NOs : 6 ou un variant de ladite séquence SEQ ID NO : 6, ledit variant présentant au moins 80% d'identité (de préférence au moins 85% et avantageusement au moins 90% d'identité) avec SEQ ID NOs : 6,
- la séquence SEQ ID NO :7 ou un variant de ladite séquence SEQ ID NO : 7, ledit variant présentant au moins 80% d'identité (de préférence au moins 85% et avantageusement au moins 90% d'identité) avec SEQ ID NO : 7, et
- la séquence SEQ ID NO : 8 ou un variant de ladite séquence SEQ ID NO : 8, ledit variant présentant au moins 80% d'identité (de préférence au moins 85% et avantageusement au moins 90% d'identité) avec SEQ ID NO : 8,
- et facultativement la séquence variable VR6 identifiée en SEQ ID NO : 9.

Ainsi, une des protéine chimérique de l'invention comprend (ou consiste essentiellement en ou consiste en) la séquence SEQ ID NO : 1, la séquence SEQ ID NO : 6, la séquence SEQ ID NO: 7, et la séquence SEQ ID NO : 8, voire de plus la séquence SEQ ID NO : 9.

Les protéines chimériques préférées de l'invention sont particulièrement identifiées comme comprenant (ou consistant essentiellement en ou consistant en) une séquence choisie parmi SEQ ID NO : 20, SEQ ID NO : 21 et SEQ ID NO : 23 ; la protéine la plus préférée étant celle qui comprend ou qui consiste en une séquence identifiée en SEQ ID NO : 20 dans le listage de séquences.

SEQ ID NO : 1 correspond à la séquence du domaine extracellulaire de VlsE de *B. garinii* (souche pBi) amputé de sa séquence signal (aa 1-19) et de la région C-terminale de la protéine mature située après le domaine IR6, c'est à dire que ce domaine extracellulaire est composé des régions IR1, VR1, IR2, VR2, IR3, VR3, IR4, VR4, IR5, VR5 et IR6 de B. *garinii* (souche pBi).

SEQ ID NO : 2 correspond à la séquence du domaine extracellulaire de VlsE de *B. garinii* (souche pBr) amputé de sa séquence signal et de la région C-terminale de la protéine mature située après le domaine IR6, c'est à dire que ce domaine extracellulaire est composé des régions IR1, VR1, IR2, VR2, IR3, VR3, IR4, VR4, IR5, VR5 et IR6 de B. *garinii* (souche pBr) .

SEQ ID NO : 3 correspond à la séquence du domaine extracellulaire de VlsE de *B. garinii* (souche pLi) amputé de sa séquence signal et de la région C-terminale de la protéine mature située après le domaine IR6, c'est à dire que ce domaine extracellulaire est composé des régions IR1, VR1, IR2, VR2" IR3, VR3, IR4, VR4, IR5, VR5 et IR6 de B. *garinii* (souche pLi).

SEQ ID NO : 4 correspond à la séquence du domaine extracellulaire de VlsE de *B. afzelli* (souche pKo) amputé de sa séquence signal et de la région C-terminale de la protéine mature située après le domaine IR6, c'est à dire que ce domaine extracellulaire est composé des régions IR1, VR1, IR2, VR2, IR3, VR3, IR4, VR4, IR5, VR5 et IR6 de B. *afzelii* (souche pKo).

SEQ ID NO : 5 correspond à la séquence du domaine extracellulaire de VlsE de *B. burgdorferi* sensu stricto (souche B31) amputé de sa séquence signal et de la région C-terminale de la protéine mature située après le domaine IR6, c'est à dire que ce domaine extracellulaire est composé des régions IR1, VR1, IR2, VR2 " IR3, VR3, IR4, VR4, IR5, VR5 et IR6 de B. *burgdorferi* sensu stricto (souche B31).

SEQ ID NO : 6 correspond à la séquence du domaine IR6 de *B. burgdorferi* sensu stricto (souche B31).

SEQ ID NO : 7 correspond à la séquence du domaine IR6 de *B. afzelii* (souche ACA-1).

SEQ ID NO : 8 correspond à la séquence du domaine IR6 de *B. garinii* (souche Ip90).

SEQ ID NOs : 9 correspond à la séquence de la région variable VR6 de *B. burgdorferi* sensu stricto (souche B31). Cette séquence peut être introduite dans la construction en tant que bras espaceur entre les domaines IR6.

Une séquence d'au moins 6 Histidine (queue polyhistidine identifiée en SEQ ID NO : 10, codée par l'une quelconque des séquences nucléiques identifiées en SEQ ID NOs 11, 12 et 13, peut être ajoutée au niveau de l'extrémité N-terminale ou C-terminale de la protéine pour permettre sa purification sur résine métal-chélate, ainsi que des acides aminés additionnels représentés en SEQ ID NO : 14 et codés par la séquence SEQ ID NO : 15 en amont de la queue polyhistidine. Dans cette configuration la protéine comprend ou consiste en une séquence identifiée comme SEQ ID NO : 21. Alternativement, une séquence de 8 Histidine représentée en SEQ ID NO : 16 et codée par SEQ ID NO : 17 peut être placée en N-terminal de la protéine à la place de la séquence de 6 Histidine ce qui permet de stabiliser l'accrochage de la protéine recombinante sur la résine métal-chélate et d'améliorer les conditions de purification ainsi que des acides aminés additionnels représentés en SEQ ID NO : 18 et codés par la séquence SEQ ID NO : 19. Dans cette configuration, la protéine comprend ou consiste en une séquence identifiée comme SEQ ID NO :23.

Les protéines préférées de l'invention sont celles identifiées comme SEQ ID NOs : 21 et 23, respectivement codées par les séquences SEQ ID NOs : 22 et 24.

L'invention a également pour objet les séquences ADN codant les protéines telles que définies précédemment et en particulier les séquences identifiées SEQ ID NOs : 22 et 24.

L'invention a aussi pour objet une cassette d'expression qui est fonctionnelle dans une cellule dérivée d'un organisme procaryote (exemple : *Escherichia coli*) ou eucaryote, tel qu'une levure (exemple : *Pichia, Schizosaccharomyces*) permettant l'expression de l'acide nucléique décrit ci-dessus (ADN), lorsqu'elle est placée sous le contrôle des éléments permettant son expression, ainsi que le vecteur comprenant une telle cassette.

La protéine de l'invention est notamment utilisable pour le diagnostic d'une infection à borrélia. Ainsi, la présente invention a pour objet un procédé pour le diagnostic *in vitro* d'une Borreliose de Lyme dans un échantillon biologique (par exemple un échantillon de sérum, de sang, de plasma etc...), selon lequel on met en contact l'échantillon biologique avec au moins une protéine telle que définie ci-dessus et on détermine si il y a formation d'un complexe immunologique entre ladite protéine et des anticorps de l'échantillon biologique (IgGs et/ou IgMs), par exemple par addition d'au moins une anti-immunoglobuline humaine marquée par tout marqueur approprié. Par marqueur, on entend un traceur capable d'engendrer un signal. Une liste non limitative de ces traceurs comprend les enzymes qui produisent un signal détectable par exemple par colorimétrie, fluorescence ou luminescence, comme la peroxydase de raifort, la phosphatase alcaline, la β-galactosidase, la glucose-6-phosphate déshydrogénase ; les chromophores comme les composés fluorescents, luminescents ou colorants ; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme ³²P, ³⁵S ou ¹²⁵I. De préférence, la protéine est immobilisée sur un support solide qui peut être le cône d'un appareil Vidas@, les puits d'une plaque de microtitration, une particule, un gel etc...

Dans un mode de réalisation de l'invention, l'échantillon est de plus mis en contact avec au moins une protéine de fusion chimérique choisie parmi celles décrites ci-dessous :
(a) une protéine dont la séquence en acides aminés comprend (ou consiste en) la séquence SEQ ID NO : 25 et la séquence SEQ ID NO : 26 ou une séquence qui présente au moins 40% d'identité avec SEQ ID NO : 25 et une séquence qui présente au moins 50% d'identité avec SEQ ID NO : 26,
(b) une protéine dont la séquence en acides aminés comprend (ou consiste en) la séquence SEQ ID NO : 27 et la séquence SEQ ID NO : 28 ou une séquence qui présente au moins 40% d'identité avec SEQ ID NO : 27 et une séquence qui présente au moins 50% d'identité avec SEQ ID NO : 28,
(c) une protéine, dont la séquence en acides aminés comprend (ou consiste en) une séquence choisie parmi :
   (i) la séquence SEQ ID NO : 29 et la séquence SEQ ID NO : 31 ou une séquence qui présente au moins 40% d'identité avec SEQ ID NO : 29 et une séquence qui présente au moins 50% d'identité avec SEQ ID NO : 31,
   (ii) la séquence SEQ ID NO : 30 et la séquence SEQ ID NO : 31 ou une séquence qui présente au moins 40% d'identité avec SEQ ID NO : 30 et une séquence qui présente au moins 50% d'identité avec SEQ ID NO : 31
   (iii) la séquence SEQ ID NO 29, la séquence SEQ ID NO : 30 et la séquence SEQ ID NO : 31, ou une séquence qui présente au moins 40% d'identité avec SEQ ID NO : 29, une séquence qui présente au moins 40% d'identité avec SEQ ID NO : 29 et une séquence qui présente au moins 50% d'identité avec SEQ ID NO : 31
(d) une protéine dont la séquence en acides aminés comprend (ou consiste en) une séquence choisie parmi SEQ ID NOs : 32, 34, 36 ou une séquence choisie parmi SEQ ID NOs : 33, 35, 37 et 38 décrites plus en détail ci-dessous.

Chacune des protéines identifiées ci-dessus comprend au moins une séquence du domaine extracellulaire d'une protéine DbpA d'une espèce de Borrelia choisie parmi *B. afzelii* (SEQ ID NO : 25), *B. burgdorferi* sensu stricto (SEQ ID NO : 27) et B. *garinii* (groupe III : SEQ ID NO : 29) (groupe IV : SEQ ID NO : 30) ou une séquence présentant au moins 40% d'identité avec lesdites séquences et au moins une séquence d'une protéine OspC de *B. afzelii* (SEQ ID NO : 26), *B. burgdorferi* sensu stricto (SEQ ID NO : 28) et *B. garinii* (SEQ ID NO : 31) ou une séquence qui présente au moins 50% d'identité avec lesdites séquences. Préférentiellement, la (les) séquence(s) DbpA est (sont) placée(s) côté N-terminal de la protéine recombinante et la séquence OspC est placée côté C-terminal de la protéine recombinante.

Comme décrit précédemment, une séquence d'au moins 6 Histidine peut être ajoutée au niveau de l'extrémité N-terminale ou C-terminale de la protéine pour permettre sa purification sur résine métal-chélate. La séquence de 6 histidine, identifiée en SEQ ID NO : 10, est placée préférentiellement côté N-terminal de la construction. Des acides aminés additionnels peuvent être présents en amont de la queue poly-His du fait de l'insertion dans la séquence d'ADN codante d'une petite séquence qui permet de faciliter le clonage de la séquence d'intérêt dans le plasmide d'expression, par exemple le motif « MRGS » (SEQ ID NO : 14) codé par ATGAGGGGATCC (SEQ ID NO : 15).

Une région de liaison, peut être introduite entre chacune des séquences DbpA et OspC qui compose une protéine recombinante chimérique. Ce type de région correspond à une région d'espacement, flexible, assurant une meilleure accessibilité des anticorps potentiels à chacun des domaines. Elle est généralement riche en acides aminés Gly et Ser, acides aminés décrits comme apportant une flexibilité dans la structure tertiaire de la protéine. Il est aussi possible d'introduire dans une séquence d'intérêt codante un bras ADN (ou linker) pour favoriser la liaison entre les séquences codantes pour deux protéines d'intérêt. C'est par exemple le motif « GSGG » (SEQ ID NO : 46) codé par la séquence GGTTCCGGGGGT (SEQ ID NO : 47) qui agit comme bras de liaison entre les protéines DbpA groupe IV et OspC de *B. garinii.*

Des exemples de ces protéines sont représentés par les SEQ ID NOs : 33, 35, 37 et 38 dans le listage de séquences.

Les protéines décrites ci-dessus et identifiées comme SEQ ID NOs : 32 à 38 dans le listage de séquences sont respectivement codées par les séquences ADN correspondantes identifiées en SEQ ID NOs : 39 à 45.

L'invention a encore pour objet une trousse pour le diagnostic *in vitro* d'une Borréliose de Lyme comprenant au moins une protéine VlsE chimère telle que décrite ci-dessus et facultativement au moins une protéine de fusion chimérique DbpA/OspC telle que définie précédemment, et de préférence comprenant au moins une anti-immunoglobuline humaine marquée par tout marqueur approprié répondant aux définitions données précédemment.

### Exemple

### Exemple 1 : Préparation des constructions plasmidiques codants pour les protéines recombinantes chimériques VlsE

Les séquences ADN codant les différentes séquences de la protéine sont identifiées dans le tableau 1.

**Tableau 1 : Origine des séquences**

| | **Espèces de *B. burgdorferi *Isolat ; **acides aminés (aa) ; ***N° d'accession GenBank*** | | |
|---|---|---|---|
| protéine | B. sensu stricto | B. *afzelii* | *B. garinii* |
| VlsE | - | - | *PBi ; **aa 20-293; ***AJ630106 (GenScript Corp) |
| IR6 | *B31; **aa 274-305; ***U76405 (GeneArt GmbH) | *ACA-1; **aa 172-188; ***U76905 (GeneArt GmbH) | *Ip90; **aa 167-191; ***AAN87834 (GeneArt GmbH) |

Les séquences ont été optimisées pour leur expression dans E. Coli en utilisant GeneOptimizer™ et synthétisées respectivement par GenScript corporation (Scotch Plains, NJ, USA) ou GeneArt GmbH (Regensburg, Alemagne).

Des modifications supplémentaires à l'ADN, délétions ou associations de différentes séquences ont été réalisées par PCR par génie génétique en utilisant les techniques de PCR bien connues de l'homme du métier et décrites par exemple dans Sambrook J. et al., Molecular Cloning : A Laboratory Manual, 1989.. Les séquences d'ADN ont été liées dans le vecteur d'expression pMR [2] ou pET-3d (Novagen®). Les constructions plasmidiques et les protéines correspondantes citées en exemple (bLYM110, bLYM125) sont décrites dans le tableau 2.

**Tableau 2: Constructions plasmidiques et protéines correspondantes**

| Caractéristiques des protéines recombinantes | | | Caractéristiques des constructions plasmidiques | |
|---|---|---|---|---|
| Nom | N-terminal Tag | Séquence *B. burgdorferi* | Vecteur parental | *Site d'insertion dans le vecteur* de *la sequence insert* |
| bLYM110 SEQ ID NO : 21 | 6 x His | VlsE *garinii* pBi aa 20-293 + 3 IR6 [sensu stricto B21 aa *274-305 + afzelii* ACA-1aa 172-188 + *garinii* Ip90 aa 167-191] | pMR78 | *5'BamHI* / *3'HindIII* |
| bLYM125 SEQ ID NO: 23 | 8 x His | | pET-3d | *5'NcoI* / *3'BamHI* |

### Exemple 2 Expression des protéines recombinantes de l'exemple 1 et purification.

Une construction plasmidique décrite dans l'exemple 1 a été utilisée pour transformer une bactérie E. *coli* (souche BL21) selon un protocole classique connu de l'homme du métier. Les bactéries transformées ont été sélectionnées grâce à leur résistance à l'ampicilline portée par le vecteur pMR ou pET.

Un clone de bactérie recombinante a alors été sélectionné pour ensemencer une pré-culture de 40 ml de milieu 2xYT (tryptone 16 g/l ; extrait de levure 10 g/l ; NaCl 5 g/l, pH 7,0) contenant 100 µg/ml ampicilline. Après 15 à 18 heures d'incubation à 30°C sous agitation à 250 rpm, cette pré-culture a été utilisée pour ensemencer 1 litre de milieu 2xYT contenant 2 % glucose et 100 µg/ml ampicilline. Cette culture a été incubée à 30°C sous agitation à 250 rpm jusqu'à ce que la DO à 600 nm atteigne 1,0/1,2. La culture a été maintenue pendant 3 heures 30 min. ou 4 heures à 30°C en ajoutant de l'isopropyl-β-D-thiogalactopyranoside (IPTG) 0.4mM et récoltée par centrifugation à 6000 g pendant 30 min. Le culot cellulaire a été stocké à -60°c. Pour la purification, la biomasse humide a été re-suspendue dans un tampon de lyse contenant des inhibiteurs des protéases sans EDTA (Roche) et de la benzonase nuclease (Novagen®) et soumise à une rupture cellulaire à 1,6 kBar dans un destructeur de cellules (Constant System Ltd, Daventry, ROYAUME-UNI). Le lysat a été alors centrifugé à 10,000 rpm. pendant 45 minutes à 2-8°C. Après filtration sur un filtre de 0.22 µm, le surnageant a été chargé sur une colonne NiNTA (Quiagen®) équilibrée dans un tampon de lyse. La résine a été alors lavée avec le même tampon jusqu'à ce que l'A₂₈₀ₙₘ ait atteint la ligne de base. Une élution a été effectuée avec le tampon d'élution et la protéine purifiée a été dialysée sur une Cassette Pierce de dialyse Slide-A-Lyser® 10000 ou 20000 MWCO contre le tampon de dialyse. Les conditions de purifications sur gel Ni-NTA sont décrites dans le tableau 3.

**Tableau 3: purification des protéines recombinantes**

| Protéine | bLYM110 SEQ ID NO : 21 | bLYM125 SEQ ID NO : 23 |
|---|---|---|
| Tampon de lyse et de lavage | Tampon A¹ | Tampon A¹+ Urée 2M |
| Tampon d'élution | Tampon B ² | Tampon B ² modifié avec 600 mM Imidazole |
| Elution étape 1 | 86% Tampon A + 14% Tampon B (4CV) | 92% Tampon A + 8% Tampon B (4CV) |
| Elution étape 2 | 100 % Tampon B | 100 % Tampon B |
| Rendement de purification mg protéine /g biomasse humide | 0, 5 | 0,8 |
| Rendement de purification mg protéine /L de culture | 8,7 | 17 |
| ¹Sodium phosphate 50 mM, Imidazole 30 mM, NaCl 500 mM, Tween 20 0,1 %, Glycerol 5 %, pH = 7,8 | | |
| ²Sodium phosphate 50 mM, Imidazole 325 mM, NaCl 500 mM, Glycerol 5 %, pH = 7,5 | | |

Les échantillons ont été analysés sur NuPAGE® Novex® 4-12% dans un tampon circulant NuPAGE® MES-SDS, selon les instructions du fabricant (Invitrogen™). Les protéines ont été soit colorées au bleu brillant de Coomassie ou ont été transférées électrophorètiquement sur une membrane de nitrocellulose. La membrane a été bloquée avec du lait sec 5% (w/v) dans du PBS et incubée avec un anticorps anti-pentahistidine (Qiagen®) dans du PBS contenant du Tween 20 0,05%. Un conjugué IgG anti-souris de chèvre marqué à la peroxydase de raifort (Jackson Immunoresearch laboratories) dans du PBS/Tween a été utilisé comme anticorps secondaire.

La détermination de la concentration en protéines a été effectuée en utilisant le Bradford Assay Kit (Pierce Coomassie Plus, Perbio Science) avec la BSA comme standard protéique.

### Exemple 3 : Détection des IgGs et des IgMs humaines avec la protéine recombinante chimérique bLYM110 de l'exemple 2 par une technique Line Immunoblot

la protéine recombinante a été déposée sur une membrane de difluorure de polyvinylidene (PVDF, Immobilon, Millipore®, Bedford, Mass. USA) selon le protocole suivant :
La concentration en protéine a été ajustée à 1 mg/ml dans du PBS pH 7.2 et diluée dans le PBS pH 7.2 additionné de Tween 20 0,03% (dilution 1/200^{ème}). La membrane PVDF a été humidifiée dans du méthanol, lavée dans de l'eau déminéralisée et couchée sur un papier de blot humide. Une règle en plastique a été plongée dans la dilution de protéines et fixée sur la membrane PVDF. Après dépôt des protéines et séchage des membranes, les membranes ont été coupées verticalement en bandelettes. Avant utilisation, les bandelettes ont été incubées avec de la gélatine 5% dans du TBS pH 7,5 pendant 1 heure à 37°C. Les protocoles d'immunoblot ont été réalisés à température ambiante comme décrit par Bretz A.G. et al. [3]. Les bandelettes ont été incubées pendant 2 heures avec des sérums humains dilués au 1/200^{ème} dans du TBS avec de la gélatine 1%, lavées et incubées avec des IgG ou IgM anti-humaines marquées à la phosphatase alcaline (Sigma™, St-Louis, USA) diluées au 1/1000^{ème} dans du TBS avec de la gélatine 1%. Après lavage, les bandelettes ont été incubées avec le substrat BCIP-NBT (KPL, Gaithersburg, MD, USA) pendant 30 minutes, lavées dans de l'eau distillée et séchées.

### Panel de sérums testés

Les sérums humains ont été collectés à partir de patients LB typiques bien définis cliniquement correspondant aux différent stades de LB (22 avec un érythème migrant [EM], 5 avec une cardite, 20 avec une neuroborreliose [NB], 20 avec de l'arthrite de Lyme [LA], 20 avec une acrodermatite chronica athrophicans [ACA] et 10 avec un lymphocytome cutis benigna [LCB]). Des IgGs anti-Lyme ont été trouvés par immunoblot, décrit précédemment utilisant des lysats de cellules entières [4], dans les sérums de patients avec LA, ACA et cardite. EM, NB et LCB ont été identifiés cliniquement, mais tous les sérums correspondants n'ont pas été trouvés positifs par l'immunoblot [4], ni par les kits disponibles commercialement (VIDAS® Lyme (biomérieux®), Borrelia IgG, (Diasorin®) et Borrelia IgM (r-biopharm®)). Par contre, tous les cas de NB inclus dans l'étude avaient des anticorps détectables dans le liquide céphalo-rachidien [LCR] (index s'étendant de 2 à 27,1) .

Le groupe contrôle négatif consistait en 31 sérums antérieurement trouvés comme étant négatifs pour la présence d'anticorps anti-Lyme dans les essais classiques. De plus, 64 sérums de donneurs de sang sains résidant dans une région endémique pour Lyme (Monthley, Valis, Suisse) ont été testés avec la protéine recombinante. L'intensité de la réaction a été évaluée comme suit : [+], [++], [+++], [-] ou résultats équivoques. Les résultats équivoques ont été considérés comme négatifs.

Les résultats sont présentés dans le tableau 4 ci-dessous.

**Tableau 4**

| **IgG** | | | | | | |
|---|---|---|---|---|---|---|
| **Stade I** | **Stade II** | | **Stade III** | | | **Donneurs** |
| EM | NB | Cardite | LA | ACA | Lymph. | |
| (n=22) | (n=20) | (n=5) | (n=19) | (n=20) | (n=10) | (n=64) |
| 17 | 20 | 5 | 19 | 20 | 9 | 6 |
| 77,3% | 100% | 100% | 100% | 100% | 90% | 9,4% |
| 12 [+++] | 11 [+++] | 4 [+++] | 13 [+++] | 20 [+++] | 3 [+++] | 6 [+] |
| 4 [++] | 7 [++] | 1 [++] | 4 [++] | | 2 [++] | |
| 1 [+] | 2 [+] | | 2 [+] | | 4 [+] | |
| **Total positifs IgG 93,7%** | | | | | | |

| **IgM** | | | | | | |
|---|---|---|---|---|---|---|
| EM | NB | Cardite | | | | |
| (n=22) | (n=20) | (n=5) | | | | (n=64) |
| 5 | 4 | 2 | | | | 1 |
| 22% | 20% | 40% | | | | 1, 5% |
| 1 [++] | 2[++] | 1 [++] | | | | 1 [+] |
| 4 [+] | 1 [+] | 1 [+] | | | | |
| **Total positifs IgM 23,4%** | | | | | | |

### Détection des IgGs

Les résultats indiquent que la protéine recombinante bLYM110 est un antigène de diagnostic hautement sensible à tous les stades de l'infection pour les IgGs. Au stade I de l'infection les IgG ont été détectées dans 17 cas des patients avec un EM sur 22 (soit 77,3% de sensibilité). Cinq des patients avec EM qui sont trouvés négatifs avec la protéine recombinante le sont également avec l'immunoblot maison et avec les kits disponibles dans le commerce. Sept sérums EM trouvés positifs avec la protéine recombinante n'étaient pas détectés en immunoblot, ce qui représente une amélioration de 31,8% de la sensibilité avec la protéine recombinante. Au stade primaire de l'infection, en l'absence de rougeur caractéristique, le diagnostic peut être difficile puisque les autres manifestations cliniques de la maladie de Lyme ne sont pas spécifiques. De plus, seulement quelques patients ayant un EM sont détectés par les tests classiques. Donc, la protéine de l'invention améliore la détection des IgGs au stade I de l'infection en portant leur détection à plus de 77% chez les patients présentant un EM.

### Détection des IgMs

Des IgMs anti-protéine chimère sont trouvés dans 23,4% des sérums LB. La protéine détecte plus souvent les IgGs que les IgMs dans les sérums de patients LB aux stades I et II.

### Exemple 4: préparation des constructions plasmidiques codants pour les protéines recombinantes chimériques DpbA-OspC.

Les séquences ADN codant pour les différentes séquences DpbA et OspC décrites sont identifiées dans le tableau 5. Les séquences ADN ont été optimisées pour favoriser l'expression dans E. Coli en utilisant GeneOptimizer™ et synthétisées respectivement par GenScript corporation (Scotch Plains, NJ, USA) ou GeneArt GmbH Regensburg, Allemagne).

**Tableau 5 : origine des séquences**

| | **Espèces de *B. burgdorferi*** | | |
|---|---|---|---|
| | **Isolat;* **acides-aminés (aa); ***N° d'accession GenBank | | |
| protéine | B. sensu stricto | *B. afzelii* | *B. garinii* |
| DbpA | *B31; **aa 2-192; ***AF069269 | *PKo; **aa 2-150; ***AJ131967 | *40; **aa 2-187; ***AF441832 *PBi; **aa 2-176 ; ***AJ841673 |
| OspC | *B31; **aa 26-210; ***X73622 | *PKo; **aa 2-212; ***X62162 | *PEi ; **aa 32-208; ***A749866 |

Chaque protéine recombinante chimérique comprend au moins un région épitopique correspondant au domaine extracellulaire d'une séquence DbpA de Borrelia burgdorferi sensu stricto ou *B. afzelii* ou *B. garinii* et au moins une région épitopiques correspondant au domaine extracellulaire d'une séquence OspC de Borrelia burgdorferi sensu stricto ou *B. afzelii* ou *B*. *garinii.*

Les associations de différentes séquences nucléotidiques codant pour des séquences DbpA et/ou OspC ainsi que les modifications de séquences nucléotidiques, telles que délétions, addition de séquence de liaison ou addition de séquence linker ont été réalisées par génie génétique en utilisant les techniques de PCR bien connues de l'homme du métier et décrites par exemple dans Sambrook J. et al., Molecular Cloning : A Laboratory Manual, 1989.

Les séquences d'ADN codant pour les protéines chimériques d'intérêt ont été introduites dans le vecteur d'expression pMR [2] entre le site de restriction BamHI en 5' et le site EcoRI ou HindIII en 3'. Les constructions plasmidiques et les protéines correspondantes citées en exemple (bLYM114, bLYM120 et bLYM121) sont décrites dans le tableau 6. La présence de MRGS en N-terminal des protéines recombinantes et la séquence nucléotidique ATG AGG GGA TCC correspondante a été introduite par la technique de clonage utilisée dans le vecteur d'expression pMR. Seul le codon d'initiation ATG et par conséquent l'acide aminé Met est vraiment indispensable dans cette séquence.

Une séquence poly-His (6xhistidine) a été introduite côté N-terminal de chaque protéine recombinante. Cette séquence permet la purification des protéines recombinantes sur colonne d'affinité métal-chélate. C'est une région de fixation sur le gel Ni-NTA qui permet de faciliter ultérieurement l'étape de purification de la protéine recombinante chimérique. Ce peptide HHHHHH (SEQ ID NO : 10) est codé par les séquences nucléotidiques CATCATCATCATCATCAT (SEQ ID NO : 11) ou CATCATCATCATCATCAC (SEQ ID NO : 12) ou CATCATCACCACCATCAT (SEQ ID NO : 13) ou par toute autre séquence codant pour la séquence SEQ ID NO : 10.

A titre indicatif, cette région de fixation particulière, comprenant une succession d'histidine, permet notamment la fixation orientée de la protéine recombinante sur un support constitué de silice ou d'oxydes métalliques.

**Tableau 6: Constructions plasmidiques et protéines correspondantes**

| Caractéristiques des protéines recombinantes | | | Caractéristiques des constructions plasmidiques | | |
|---|---|---|---|---|---|
| Nom | N-terminal Tag | Séquence *B. burgdorferi* | nom | Vecteur parental | Site *d'insertion dans le vecteur de la séquence insert* |
| bLYM114 SEQ ID NO: 33 | 6 x His | *B. afzelii* strain PKo DbpA aa 2-150 + OspC aa 2-212 | pOL114 | pMR78* | *5'BamHI* / *3'EcoRI* |
| bLYM120 SEQ ID NO : 35 | 6 x His | B.sensu stricto strain B31 DbpA aa 28-192 + OspC aa 26-210 | pOL120 | pMR78* | *5'BamHI* / *3'HindIII* |
| bLYM121 SEQ ID NO : 38 | 6 x His | *B. garinii* DbpA III aa 25-187 strain 40 + DbpA IV aa 24-176 strain PBi + OspC aa 32-208 strain PEi | pOL121 | pMR78* | *5'BamHI* / *3'HindIII* |

| | | | | | |
|---|---|---|---|---|---|
| • [2] | | | | | |

### Exemple 5 : Expression des protéines recombinantes bLYM114, bLYM120 et bLYM121 de l'exemple 4 et purification.

Une construction plasmidique dans laquelle une séquence SEQ ID NO : 40, 42 ou 45 a été insérée dans un vecteur d'expression (pMR) a été utilisée pour transformer une bactérie *E. coli* (souche BL21) selon un protocole classique connu de l'homme du métier. Les bactéries transformées ont été sélectionnées grâce à leur résistance à l'ampicilline portée par le vecteur pMR.

Un clone de bactérie recombinante a alors été sélectionné pour ensemencer une pré-culture de 40 ml de milieu 2xYT (tryptone 16 g/l ; extrait de levure 10 g/l ; NaCl 5 g/l, pH 7,0) contenant 100 µg/ml ampicilline. Après 15 à 18 heures d'incubation à 30°C sous agitation à 250 rpm, cette pré-culture a été utilisée pour ensemencer 1 litre de milieu 2xYT contenant 2 % glucose et 100 µg/ml ampicilline. Cette culture a été incubée à 30°C sous agitation à 250 rpm jusqu'à ce que la DO à 600 nm atteigne 1,0/1,2. La culture a été maintenue pendant 3 heures 30 min. ou 4 heures à 30°C en ajoutant de l'isopropyl-β-D-thiogalactopyranoside (IPTG) 0.4mM et récoltée par centrifugation à 6000 g pendant 30 min. Le culot cellulaire a été stocké à -60°C. Pour la purification, la biomasse humide a été décongelée et re-suspendue dans un tampon de lyse contenant des inhibiteurs des protéases sans EDTA (Roche™) et de la benzonase nuclease (Novagen) et soumise à une rupture cellulaire à 1,6 kBar dans un destructeur de cellules (Constant System Ltd, Daventry, ROYAUME-UNI). Le lysat a été ensuite centrifugé à 10,000 rpm. pendant 45 min. à 2-8°C. Le surnageant obtenu contient les protéines solubles. Ce surnageant a été filtré sur filtre 0,45 µ et purifié par chromatographie d'affinité sur une colonne de chélation de métaux (matrice nickel-nitrilotriacetic acid (Ni-NTA, Qiagen). Pour ce faire, le surnageant a été déposé (1 ml/min) à 18-25°C sur une colonne de 8 ml de gel Ni-NTA équilibrée en tampon A (voir tableau 7). La colonne a été ensuite lavée en tampon A, jusqu'à obtention en sortie de colonne d'une D0₂₈₀ₙₘ=0. L'élution de la protéine recombinante est obtenue par application d'un tampon B, suivant les indications reportées dans le tableau 7, et la protéine purifiée a été dialysée en cassette de dialyse 10000 ou 20000 MWCO (Slide-A-Lyser®, Pierce) contre un tampon de dialyse. Les conditions de purifications sur gel Ni-NTA sont décrites dans le tableau 7.

**Tableau 7: purification des protéines recombinantes**

| Protéine | bLYM114 | bLYM120 | bLYM121 |
|---|---|---|---|
| Tampon de lyse et de lavage | Tampon A ¹ | | |
| Tampon d'élution | Tampon B ² | | |
| Elution étape 1 | 90% Buffer A + 10% Buffer B (4CV) | 92% Buffer A + 8% Buffer B (4CV) | 100 % Buffer B |
| Elution étape 2 | 100 % Buffer B | 100 % Buffer B | NA |
| Rendement de purification mg protéine /g biomasse humide | 12 | 13 | 20 |
| Rendement de purification mg protéine /L de culture | 80 | 122 | 245 |
| ¹Sodium phosphate 50 mM, Imidazole 30 mM, NaCl 500 mM, Tween 20 0,1 %, Glycerol 5 %, pH = 7,8 | | | |
| 2 Sodium phosphate 50 mM, Imidazole 325 mM, NaCL 500 mM, Glycerol 5 %, pH = 7,5 | | | |

Les échantillons ont été analysés sur NuPAGE® Novex®4-12% dans un tampon NuPAGE® MES-SDS, selon les instructions du fabricant (Invitrogen). Les protéines ont été soit colorées au bleu brillant de Coomassie ou ont été transférées électrophorètiquement sur une membrane de nitrocellulose. La membrane a été bloquée avec du lait sec 5% (w/v) dans du PBS et incubée avec un anticorps anti-pentahistidine (Qiagen®) dans du PBS contenant du Tween 20 0,05%. Un conjugué IgG anti-souris de chèvre marqué à la peroxydase de raifort (Jackson Immunoresearch laboratories) dans du PBS/Tween a été utilisé comme anticorps secondaire.

La détermination de la concentration en protéines a été effectuée en utilisant le kit Bradford (Pierce Coomassie Plus, Perbio Science) avec la BSA comme standard protéique.

### Exemple 6 : Détection des IgGs et des IgMs humaines avec les protéines recombinantes chimériques par une technique Line Immunoblot

Chaque protéine recombinante a été déposée sur une membrane de difluorure de polyvinylidene (PVDF, Immobilon, Millipore, Bedford, Mass. USA) selon le protocole suivant :
La concentration en protéine a été ajustée à 1 mg/ml dans du PBS pH 7.2 et diluée dans le PBS pH 7.2 additionné de Tween 20 0,03% (dilution 1/200^{ème}). La membrane PVDF a été humidifiée dans du méthanol, lavée dans de l'eau déminéralisée et couchée sur un papier de blot humide. Une règle en plastique a été plongée dans la dilution de protéines et fixée sur la membrane PVDF. Après dépôt des protéines et séchage des membranes, les membranes ont été coupées verticalement en bandelettes. Avant utilisation, les bandelettes ont été incubées avec de la gélatine 5% dans du TBS pH 7,5 pendant 1 heure à 37°C. Les protocoles d'immunoblot ont été réalisés à température ambiante comme décrit par Bretz A.G. et al. [3]. Les bandelettes ont été incubées pendant 2 heures avec des sérums humains dilués au 1/200^{ème} dans du TBS avec de la gélatine 1%, lavées et incubées avec un anticorps anti-IgG ou anti-IgM humaines marqué à la phosphatase alcaline (Sigma, St-Louis, USA) diluées au 1/1000^{ème} dans du TBS avec de la gélatine 1%. Après lavage, les bandelettes ont été incubées avec le substrat BCIP-NBT de la phosphatase alcaline (KPL, Gaithersburg, MD, USA) pendant 30 min., puis lavées dans de l'eau distillée et séchées.

### Panel de sérums testés

Les sérums humains ont été collectés à partir de patients LB typiques bien définis cliniquement correspondant aux différent stades de LB (22 avec un érythème migrant [EM], 5 avec une cardite, 20 avec une neuroborreliose [NB], 20 avec de l'arthrite de Lyme [LA], 20 avec une acrodermatite chronica athrophicans [ACA] et 10 avec un lymphocytome cutis benigna [LCB]). Des IgGs anti-Lyme ont été trouvés par immunoblot,]utilisant des lysats de cellules entières [4], dans les sérums de patients avec LA, ACA et cardite. EM, NB et LCB ont été identifiés cliniquement, mais tous les sérums correspondants n'ont pas été trouvés positifs par l'immunoblot [4], ni par les kits disponibles commercialement (VIDAS® Lyme (biomérieux), Borrelia IgG, (Diasorin®) et Borrelia IgM (r-biopharm®). Par contre, tous les cas de NB inclus dans l'étude avaient des anticorps détectables dans le liquide céphalo-rachidien [LCR] (index s'étendant de 2 à 27,1 par VIDAS@ Lyme (biomérieux).La présence d'IgM n'a été recherchée que dans les cas cliniques de stade I et II et pas dans les stades chroniques.

Le groupe contrôle négatif consistait en 31 sérums antérieurement trouvés comme étant négatifs pour la présence d'anticorps anti-Lyme dans les essais classiques. De plus, 64 sérums de donneurs de sang sains résidant dans une région endémique pour Lyme (Monthley, Valais, Suisse) ont été testés avec la protéine recombinante.

L'intensité de la réaction a été évaluée comme suit : [+], [++], [+++], [-] ou résultats équivoques. Les résultats équivoques ont été considérés comme négatifs.

Les résultats sont présentés dans le tableau 8 ci-dessous.

**Tableau 8: Réactivité en Line immunoblot des sérums humains de patients avec borréliose de Lyme avec 3 protéines recombinantes chimériques**

| Protéine recombinante | **IgG** | | | | | | **IgM** | | |
|---|---|---|---|---|---|---|---|---|---|
| | Stade I | Stade II | | Stade III | | | Stade I | Stage II | |
| | EM (n=22) | IIB (n=20 ) | Carditis (n=5) | LA (n=19 ) | ACA (n=20 ) | LCB (n=10 ) | EM (n=22) | NB (n=20 ) | Carditis (n=5) |
| bLYM114 | 5 | 10 | 0 | 7 | 12 | 2 | 7 | 7 | 2 |
| bLYM120 | 6 | 7 | 0 | 8 | 6 | 0 | 11 | 7 | 2 |
| bLYM121 | 2 | 10 | 5 | 9 | 8 | 0 | 7 | 7 | 2 |
| Σ bLYM 114+120+121 | 9 | 13 | 5 | 18 | 17 | 2 | 11 | 7 | 2 |
| serums positifs (%) et Intensité de la réaction | **40.9%** | **59.1%** | **100%** | **94.7%** | **85%** | **20%** | **50%** | **35%** | **40%** |
| | 1 [+++] | 8[+++] | 4[+++] | 7[+++] | 8[+++] | | 1 [+++] | | |
| | 4 [++] | | | | | 1[++] | 7 [++] | 5 [++] | 2 [++] |
| | 4 [+] | 2 [++] | 1 [+] | 8[++] | 5 [++] | 1 [+] | 5 [+] | 2 [+] | |
| | | 3 [+] | | 3 [+] | 4 [+] | | | | |
| Total positifs et intensité de la réaction | **66.7%** | | | | | | **42.5%** | | |
| | | 28 [+++] | | | | | 1 [+++] | | |
| | | 20[++] | | | | | 14 [++] | | |
| | | 16 [+] | | | | | 7 [+] | | |

La spécificité est de 100% sur la base de 31 sérums provenant de sujets sains déterminés Lyme négatifs par les tests commercialisés standards.

### Détection des IgGs

Les résultats indiquent que les protéines de fusion chimériques recombinantes sont des outils de diagnostic sensibles à tous les stades de l'infection pour les IgGs et les IgMs. Ils mettent en évidence un effet complémentaire des trois protéines recombinantes basées respectivement sur des séquences de *Borrelia afzelii*, B. sensu stricto et B. *garinii* pour la détection des IgGs. L'utilisation combinée des trois protéines recombinantes chimériques permet au stade I de l'infection de détecter des IgGs chez 9 cas des patients avec un EM sur 22 (soit 40,9% de sensibilité).

### Détection des IgMs

Des IgMs anti-protéines chimères sont trouvées dans 11 cas sur 22 (soit 50% de sensibilité). Ces protéines chimères détectent donc plus souvent les IgMs que les IgGs dans les sérums de patients LB aux stades I. Les tests effectués en contrôle : immunoblot [4], et kit commercialisé Borrelia IgM (r-biopharm®) ne détectent pas davantage de sérums IgM positifs. De plus 3 sérums trouvés négatifs par le test immunoblot et Borrelia IgM (r-biopharm®) sont détectés par les trois protéines chimériques citées en exemple (3/3) ou par une des trois protéines cités en exemple (1/3). L'utilisation combinée des trois protéines recombinantes permet d'améliorer en stade I de l'infection de 13,6% la sensibilité de détection des IgMs.

### Exemple 7 : Evaluation et validation des protéines recombinantes chimériques bLYM114, bLYM120, bLYM121 et bLYM125 dans un test VIDAS@ (bioMérieux)

Cette validation est réalisée en test VIDAS@ par l'utilisation
- 1) des protéines chimériques recombinantes bLYM114, bLYM120 et bLYM121, obtenues selon les exemples 4, 5 pour la détection des IgMs, et
- 2) des protéines chimériques recombinantes bLYM114, bLYM120, obtenues selon les exemples 4, 5 et de la protéine chimérique bLYM125, obtenue selon l'exemple 1,2 pour la détection des IgGs.

Le principe du test VIDAS® est le suivant: un cône constitue le support solide qui sert également de système de pipetage pour les réactifs présents dans la barrette. La ou les protéines recombinantes sont fixées sur le cône. Après une étape de dilution, l'échantillon est aspiré et refoulé plusieurs fois à l'intérieur du cône. Ceci permet aux immunoglobulines anti-Lyme de l'échantillon de se lier aux protéines recombinantes. Les composants non liés sont éliminés par lavage. Un anticorps anti-immunoglobulines humaines conjugué à la phosphatase alcaline (PAL) est incubé dans le cône où il se fixe aux immunoglobulines anti-Lyme. Des étapes de lavage éliminent le conjugué non fixé. Pendant la dernière étape de révélation, le substrat de la phosphatase alcaline (PAL), le 4-méthyl-umbelliferyl phosphate, est hydrolysé en 4-méthyl-umbelliferone dont la fluorescence émise à 450 nm est mesurée. L'intensité de la fluorescence est mesurée par le système optique du Vidas@ et est proportionnelle à la présence d'immunoglobulines anti-Lyme présentes dans l'échantillon. Les résultats sont analysés automatiquement par le VIDAS@ et exprimés en RFV (Relative Fluorescent Value).

255 sérums positifs (sérums équivoques + sérums positifs) et 298 sérums négatifs (équivoques + négatifs) ont ainsi été dosés avec le système Vidas@.

Les cônes Vidas@ Lyme IgG sont sensibilisés par 300 µL de solution comprenant les protéines bLYM114, bLYM120 et bLYM125 de l'invention à une concentration de 1 µg/mL chacune dans une solution de sensibilisation commune.

Dans la première étape, les sérums sont incubés pendant 5,3 min. pour la formation des complexes antigènes-anticorps. Dans la seconde étape, des anti-IgG humaines marquées à la PAL sont incubés pendant 5,3 min.

Les résultats sont donnés en indice par rapport à un seuil de positivité positionné à 135 RFV dans le protocole.
- Parmi les 255 sérums positifs testés, 246 sont positifs et 9 sont faussement négatifs, ce qui correspond à une sensibilité de 96.5 %.
- Parmi les 298 sérums négatifs testés, 284 sont négatifs et 14 sont faussement positifs, ce qui correspond à une spécificité de 95,3%.

### Références bibliographiques

1. Göttner G. et al., Int. J. Microbiol. 293, Suppl. 37, 172-173 (2004)
2. Arnaud N. et al., Gene 1997; 199:149-156.
3. Bretz A.G., K. Ryffel, P. Hutter, E. Dayer and O. Péter. Specificities and sensitivities of four monoclonal antibodies for typing of Borrelia burgdorferi sensu lato isolates. Clin. Diag. Lab. Immunol. 2001 ; 8 : 376-384.
4. Ryffel K., Péter O., Rutti B. and E. Dayer. Scored antibody reactivity by immunoblot suggests organotropism of Borrelia burgdorferi sensu stricto, B. garinii, B. afzelii and B. valaisiana in human.J. Clin. Microbiol. 1999 ; 37:4086-92
5. Steere AC. et al., Clin Infect Dis 2008; 47:188-195.

## Revendications

1. Protéine chimérique de Borrelia comprenant au moins une séquence du domaine extracellulaire d'une protéine VlsE d'une première espèce de Borrelia correspondant à une souche déterminée et au moins une séquence d'une région IR6 d'une protéine VlsE d'une seconde espèce de Borrelia ou de la première espèce de Borrelia mais correspondant à une souche différente de celle de la première espèce, ladite protéine chimérique comprenant :
- la séquence du domaine extracellulaire de la protéine VlsE de la première espèce de Borrelia est composée de cinq régions variables VR1, VR2, VR3, VR4 et VR5 et de six régions invariables IR1, IR2, IR3, IR4, IR5 et IR6, et ladite au moins une séquence du domaine extracellulaire est choisie dans le groupe consistant en SEQ ID NOs : 1, 2, 3, 4 et 5 ou un variant d'une desdites séquences SEQ ID NOs 1, 2, 3, 4 et 5, ledit variant présentant au moins 50% d'identité avec SEQ ID NOs 1, 2, 3, 4 et 5, respectivement, à la condition que ledit variant soit capable de former un complexe immunologique avec des anticorps produits suite à une infection par Borrelia, et
- ladite au moins une séquence de la région IR6 de la seconde espèce de Borrelia ou de la première espèce de Borrelia mais correspondant à une souche différente de celle de la première espèce est choisie dans le groupe consistant en SEQ ID NOs : 6, 7 et 8 ou un variant d'une desdites séquences SEQ ID NOs 6, 7, et 8, ledit variant présentant au moins 80% d'identité avec SEQ ID NOs 6, 7, et 8, respectivement, à la condition que le variant de ladite séquence soit capable de former un complexe immunologique avec des anticorps produits suite à une infection par Borrelia.

2. Protéine selon la revendication 1, comprenant de plus une séquence VR6 d'une espèce de Borrelia identifiée en SEQ ID NO : 9.

3. Protéine selon la revendication 1, comprenant :
- la séquence SEQ ID NO : 1 ou un variant de ladite séquence SEQ ID NO : 1, ledit variant présentant au moins 50% d'identité avec SEQ ID NO : 1,
- la séquence SEQ ID NOs : 6 ou un variant de ladite séquence SEQ ID NO : 6, ledit variant présentant au moins 80% d'identité avec SEQ ID NOs : 6,
- la séquence SEQ ID NO : 7 ou un variant de ladite séquence SEQ ID NO : 7, ledit variant présentant au moins 80% d'identité avec SEQ ID NO : 7, et
- la séquence SEQ ID NO : 8 ou un variant de ladite séquence SEQ ID NO : 8, ledit variant présentant au moins 80% d'identité avec SEQ ID NO : 8.

4. Protéine selon la revendication 3, comprenant de plus la séquence SEQ ID NO : 9.

5. Protéine selon la revendication 3 comprenant la séquence SEQ ID NO : 1, la séquence SEQ ID NO : 6, la séquence SEQ ID NO: 7 et la séquence SEQ ID NO : 8.

6. Protéine selon la revendication 5, comprenant la séquence SEQ ID NO : 9.

7. Protéine selon l'une quelconque des revendications précédentes comprenant une séquence choisie parmi SEQ ID NO : 20, SEQ ID NO : 21 et SEQ ID NO : 23.

8. Acide nucléique codant pour une protéine telle que définie dans l'une quelconque des revendications 1 à 7.

9. Cassette d'expression qui est fonctionnelle dans une cellule dérivée d'un organisme procaryote ou eucaryote, permettant l'expression d'un acide nucléique tel que défini dans la revendication 8, placée sous le contrôle des éléments nécessaires à son expression.

10. Vecteur comprenant une cassette d'expression, telle que définie dans la revendication 9.

11. Procédé pour le diagnostic in vitro d'une Borréliose de Lyme dans un échantillon biologique, selon lequel on met en contact l'échantillon biologique avec au moins une protéine telle que définie dans l'une quelconque des revendications 1 à 7 et on détermine si il y a formation d'un complexe immunologique entre ladite protéine et des anticorps de l'échantillon biologique.

12. Procédé selon la revendication 11, dans lequel les anticorps de l'échantillon biologique sont des IgGs et/ou des IgMs.

13. Procédé selon la revendication 11, dans lequel la formation du complexe immunologique est déterminée par addition d'au moins une anti-immunoglobuline humaine marquée par tout marqueur approprié.

14. Procédé selon l'une quelconque des revendications 11 à 13, dans lequel la protéine est immobilisée sur un support solide.

15. Trousse pour le diagnostic *in vitro* d'une Borréliose de Lyme comprenant au moins une protéine telle que définie dans l'une quelconque des revendications 1 à 7.

16. Trousse selon la revendication 15, comprenant au moins une anti-immunoglobuline humaine marquée par tout marqueur approprié.

## Patentansprüche

1. Ein chimäres Borrelien-Protein, beinhaltend mindestens eine Sequenz der extrazellulären Domäne eines VlsE-Proteins einer ersten Art von Borrellen, die einem vorherbestimmten Stamm entspricht, und mindestens eine Sequenz einer IR6-Region eines VIsE-Proteins einer zweiten Art von Borrelien oder der ersten Art von Borrelien, die allerdings einem anderen Stamm entspricht als demjenigen der ersten Art, wobei das chimäre Protein Folgendes beinhaltet:
- die Sequenz der extrazellulären Domäne des VlsE-Proteins der ersten Art von Borrelien ist aus fünf variablen Regionen VR1, VR2, VR3, VR4 und VR5 und aus sechs nicht variablen Regionen IR1, IR2, IR3, IR4, IR5 und IR6 zusammengesetzt und die mindestens eine Sequenz der extrazellulären Domäne ist ausgewählt aus der Gruppe, bestehend aus SEQ ID NO: 1, 2, 3, 4 und 5 oder einer Variante einer dieser Sequenzen SEQ ID NO 1, 2, 3, 4 und 5, wobei die Variante zu mindestens 50 % mit SEQ ID NO 1, 2, 3, 4 bzw. 5 identisch ist, vorausgesetzt, dass die Variante mit Antikörpern, die aufgrund einer Infektion mit Borrelien produziert wurden, einen immunbiologischen Komplex bilden kann, und
- die mindestens eine Sequenz der IR6-Region der zweiten Art von Borrelien oder der ersten Art von Borrelien, die allerdings einem anderen Stamm entspricht als demjenigen der ersten Art, ist ausgewählt aus der Gruppe, bestehend aus SEQ ID NO: 6, 7 und 8 oder einer Variante einer dieser Sequenzen SEQ ID NO 6, 7 und 8, wobei die Variante zu mindestens 80 % mit SEQ ID NO 6, 7 bzw. 8 identisch ist, vorausgesetzt, dass die Variante mit Antikörpern, die aufgrund einer Infektion mit Borrelien produziert wurden, einen immunbiologischen Komplex bilden kann.

2. Protein gemäß Anspruch 1, das ferner eine Sequenz VR6 einer Art Borrelien, die in SEQ ID NO: 9 identifiziert ist, beinhaltet.

3. Protein gemäß Anspruch 1, beinhaltend:
- die Sequenz SEQ ID NO: 1 oder eine Variante der Sequenz SEQ ID NO: 1, wobei die Variante zu mindestens 50 % mit SEQ ID NO: 1 identisch ist,
- die Sequenz SEQ ID NO: 6 oder eine Variante der Sequenz SEQ ID NO: 6, wobei die Variante zu mindestens 80 % mit SEQ ID NO: 6 identisch ist,
- die Sequenz SEQ ID NO: 7 oder eine Variante der Sequenz SEQ ID NO: 7, wobei die Variante zu mindestens 80 % mit SEQ ID NO: 7 identisch ist, und
- die Sequenz SEQ ID NO: 8 oder eine Variante der Sequenz SEQ ID NO: 8, wobei die Variante zu mindestens 80 % mit SEQ ID NO: 8 identisch ist.

4. Protein gemäß Anspruch 3, das ferner die Sequenz SEQ ID NO: 9 beinhaltet.

5. Protein gemäß Anspruch 3, das die Sequenz SEQ ID NO: 1, die Sequenz SEQ ID NO: 6, die Sequenz SEQ ID NO: 7 und die Sequenz SEQ ID NO: 8 beinhaltet.

6. Protein gemäß Anspruch 5, das die Sequenz SEQ ID NO: 9 beinhaltet.

7. Protein gemäß einem der vorhergehenden Ansprüche, das eine Sequenz, ausgewählt aus SEQ ID NO: 20, SEQ ID NO: 21 und SEQ ID NO: 23 beinhaltet.

8. Eine Nukleinsäure, die für ein Protein, wie in einem der Ansprüche 1 bis 7 definiert, kodiert.

9. Eine Expressionskassette, die in einer Zelle funktionell ist, die von einem prokaryonten oder eukaryonten Organismus stammt, wobei sie die Expression einer wie in Anspruch 8 definierten Nukleinsäure erlaubt, die von den zu ihrer Expression notwendigen Elementen gesteuert wird.

10. Ein Vektor, der eine Expressionskassette, wie in Anspruch 9 definiert, beinhaltet.

11. Ein Verfahren zur *In-vitro*-Diagnose einer Lyme-Borreliose in einer biologischen Probe, wobei die biologische Probe mit mindestens einem Protein, wie in einem der Ansprüche 1 bis 7 defniert, in Kontakt gebracht wird und bestimmt wird, ob sich zwischen dem Protein und Antikörpern der biologischen Probe ein immunbiologischer Komplex bildet.

12. Verfahren gemäß Anspruch 11, wobei die Antikörper der biologischen Probe IgG und/oder IgM sind.

13. Verfahren gemäß Anspruch 11, wobei die Bildung des immunbiologischen Komplexes durch die Zugabe von mindestens einem menschlichen Anti-Immunoglobulin, das mit einem (beliebigen) angemessenen Marker markiert ist, bestimmt ist.

14. Verfahren gemäß einem der Ansprüche 11 bis 13, wobei das Protein auf einem soliden Träger immobilisiert ist.

15. Ein Kit zur *In-vitro*-Diagnose einer Lyme-Borreliose, das mindestens ein Protein, wie in einem der Ansprüche 1 bis 7 definiert, beinhaltet.

16. Kit gemäß Anspruch 15, das mindestens ein menschliches Anti-Immunoglobulin, das mit einem beliebigen angemessenen Marker markiert ist, beinhaltet.

## Claims

1. A *Borrelia* chimeric protein comprising at least one sequence of the extracellular domain of a VlsE protein of a first *Borrelia* species corresponding to a determined strain and at least one sequence of an IR6 region of a VlsE protein of a second Borrelia species or of the first Borrelia species but corresponding to a strain different than that of the first species, said chimeric protein comprising:
- the sequence of the extracellular domain of the VlsE protein of the first *Borrelia* species which is composed of five variable regions VR1, VR2, VR3, VR4 and VR5 and of six invariable regions IR1, IR2, IR3, IR4, IR5 and IR6, and said at least one sequence of the extracellular domain is selected from the group consisting of SEQ ID NOs : 1, 2, 3, 4 and 5 or a variant of one of said sequences SEQ ID NOs 1, 2, 3, 4 and 5, said variant exhibiting at least 50% identity with SEQ ID NOs 1, 2, 3, 4 and 5, respectively, on the condition that said variant is capable of forming an immunological complex with antibodies produced following a Borrelia infection, and
- said at least one sequence of the IR6 region of the second Borrelia species, or of the first *Borrelia* species but corresponding to a strain different from that of the first species, which is selected from the group consisting of SEQ ID NOs : 6, 7 and 8 or a variant of one of said sequences SEQ ID NOs 6, 7 and 8, said variant exhibiting at least 80% identity with SEQ ID NOs 6, 7 and 8, respectively, on the condition that the variant of said sequence is capable of forming an immunological complex with antibodies produced following a *Borrelia* infection.

2. The protein as claimed in claim 1, comprising in addition a VR6 sequence of a Borrelia species identified in SEQ ID NO: 9.

3. The protein as claimed in claim 1, comprising:
- the sequence SEQ ID NO: 1 or a variant of said sequence SEQ ID NO: 1, said variant exhibiting at least 50% identity with SEQ ID NO: 1,
- the sequence SEQ ID NO: 6 or a variant of said sequence SEQ ID NO: 6, said variant exhibiting at least 80% identity with SEQ ID NO: 6,
- the sequence SEQ ID NO: 7 or a variant of said sequence SEQ ID NO: 7, said variant exhibiting at least 80% identity with SEQ ID NO: 7, and
- the sequence SEQ ID NO: 8 or a variant of said sequence SEQ ID NO: 8, said variant exhibiting at least 80% identity with SEQ ID NO: 8.

4. The protein as claimed in claim 3, comprising in addition the sequence SEQ ID NO: 9.

5. The protein as claimed in claim 3, comprising the sequence SEQ ID NO: 1, the sequence SEQ ID NO: 6, the sequence SEQ ID NO: 7 and the sequence SEQ ID NO: 8.

6. The protein as claimed in claim 5, comprising the sequence SEQ ID NO: 9.

7. The protein as claimed in any one of the preceding claims, comprising a sequence selected from SEQ ID NO: 20, SEQ ID NO: 21 and SEQ ID NO: 23.

8. A nucleic acid encoding a protein as defined in any one of claims 1 to 7.

9. An expression cassette which is functional in a cell derived from a prokaryotic or eukaryotic organism, allowing the expression of a nucleic acid as defined in claim 8, placed under the control of the elements necessary for its expression.

10. A vector comprising an expression cassette as defined in claim 9.

11. A method for the in vitro diagnosis of Lyme borreliosis in a biological sample, according to which the biological sample is brought into contact with at least one protein as defined in any one of claims 1 to 7 and it is determined whether there is formation of an immunological complex between said protein and antibodies of the biological sample.

12. The method as claimed in claim 11, in which the antibodies of the biological sample are IgGs and/or IgMs.

13. The method as claimed in claim 11, in which the formation of the immunological complex is determined by adding at least one anti-human-immunoglobulin labeled with any appropriate label.

14. The method as claimed in any one of claims 11 to 13, in which the protein is immobilized on a solid support.

15. A kit for the in vitro diagnosis of Lyme borreliosis comprising at least one protein as defined in any one of claims 1 to 7.

16. The kit as claimed in claim 15, comprising at least one anti-human-immunoglobulin labeled with any appropriate label.
